# EUROPÄISCHE PATENTANMELDUNG

(11) **EP 4 564 250 A1**
(43) Veröffentlichungstag der Anmeldung: **04.06.2025**
(21) Anmeldenummer: 23213484.1
(22) Anmeldetag: 30.11.2023
(51) Int. Cl.: G06Q 10/0631, G05B 19/418, G06Q 10/0633, G06Q 10/067, G06Q 10/0833, G06Q 10/087, G06Q 10/30, G06Q 50/04, A45D 34/00

(54) **VERFAHREN ZUR HERSTELLUNG EINER CHEMISCHEN ZUBEREITUNG IN EINER VERPACKUNG NEBST VERSCHLUSS, FÜR EINE CHEMISCHE ZUBEREITUNG, UMFASSEND EIN COMPUTERIMPLEMENTIERTES VERFAHREN ZUR ÜBERWACHUNG EINES ODER MEHRERER PRODUKTIONSATTRIBUTE EINES VON EINEM CHEMISCHEN PRODUKTIONSNETZWERK PRODUZIERTEN AUSGANGSMATERIALS, ENTSPRECHENDE VERFAHREN, ENTSPRECHENDE SYSTEME**

(71) Anmelder: Werner & Mertz GmbH, 55120 Mainz (DE)
(72) Erfinder: Brakemeier, Andreas, 32699 Extertal (DE); Schau, Alexander, 55128 Mainz (DE); Schlimm, Nathalie, 65203 Wiesbaden (DE); Theis, Michelle, 55576 Sprendlingen (DE)
(74) Vertreter: Eisenführ Speiser

(57) **Zusammenfassung**

Beschrieben wird ein Verfahren zur Herstellung einer chemischen Zubereitung in einer Verpackung, nebst Verschluss, vorzugsweise nebst Verschluss und Etikett, für eine chemische Zubereitung, umfassend ein computerimplementiertes Verfahren zur Überwachung eines oder mehrerer Produktionsattribute eines von einem chemischen Produktionsnetzwerk produzierten Ausgangsmaterials. Beschrieben wird zudem eine Vorrichtung zur Überwachung einer oder mehrerer Produktionseigenschaften eines Produktionsmaterials, das von einem chemischen Produktionsnetzwerk produziert wird. Beschrieben wird zudem ein Verfahren zur Herstellung mindestens eines Ausgangsmaterials, das mit einem oder mehreren Produktionsattributen verbunden ist. Beschrieben wird zudem ein System zur Herstellung mindestens eines Ausgangsmaterials, das mit einem oder mehreren Produktionsattributen verbunden ist.

## Beschreibung

Die vorliegende Erfindung betrifft ein Verfahren zur Herstellung einer chemischen Zubereitung in einer Verpackung, nebst Verschluss, vorzugsweise nebst Verschluss und Etikett, für eine chemische Zubereitung, umfassend ein computerimplementiertes Verfahren zur Überwachung eines oder mehrerer Produktionsattribute eines von einem chemischen Produktionsnetzwerk produzierten Ausgangsmaterials. Die vorliegende Erfindung betrifft zudem eine Vorrichtung zur Überwachung einer oder mehrerer Produktionseigenschaften eines Produktionsmaterials, das von einem chemischen Produktionsnetzwerk produziert wird. Die vorliegende Erfindung betrifft zudem ein Verfahren zur Herstellung mindestens eines Ausgangsmaterials, das mit einem oder mehreren Produktionsattributen verbunden ist. Die vorliegende Erfindung betrifft zudem ein System zur Herstellung mindestens eines Ausgangsmaterials, das mit einem oder mehreren Produktionsattributen verbunden ist.

### TECHNISCHES GEBIET

Die Erfindung bezieht sich auf das Gebiet der Nachhaltigkeit, insbesondere auf das Gebiet der Überwachung von Produktionsmerkmalen eines in einer chemischen Produktion hergestellten Ausgangsmaterials auf dem Gebiet der chemischen Zubereitungen, insbesondere Waschmittel und/oder Reinigungsmittel und/oder kosmetische Zubereitung, in einer Verpackung nebst Verschluss für eine chemische Zubereitung.

Die Erfindung bezieht sich auf Verfahren, Systeme, Vorrichtungen und Computerelemente zur Überwachung eines oder mehrerer Produktionsattribute mindestens eines Ausgangsmaterials, das von einem chemischen Produktionsnetzwerk hergestellt wird.

### TECHNISCHER HINTERGRUND

Chemische Produktionsnetzwerke sind groß angelegte Produktionsnetzwerke, die tausende von chemischen Produkten durch chemische Umwandlung produzieren. Mit der Transformation zur Kreislaufwirtschaft unterliegen die Eingangsmaterialien für chemische Produktionsnetzwerke dynamischen Veränderungen. Es besteht ein Bedarf an Methoden, die der Dynamik der Eingangsmaterialien Rechnung tragen und auch mit wechselnden Eingangsmaterialien zu möglichst gleichbleibenden Eigenschaften von Ausgangsmaterialien führen. Die Überwachung von Einsatzstoffen wird daher zunehmend komplexer und es besteht ein Bedarf an Methoden zur Überwachung von Eingangsstoffen, insbesondere wenn diese nicht oder nur mit einem unvorteilhaft großen Aufwand durch geeignete Analysemethoden durch ein komplexes chemisches Produktionsnetzwerk verfolgt werden können. Die Ermittlung von Ausgangsmaterialidentifikatoren betreffend die Gesamtheit einer chemischen Zubereitung und ihrer Verpackung nebst Verschluss vorzugsweise nebst Verschluss und Etikett, wird zunehmend komplexer und ist regelmäßig mit herkömmlichen Methoden nicht realisierbar. Es besteht daher ein Bedarf an Methoden zur Ermittlung entsprechender Ausgangsmaterialidentifikatoren.

Alle hierin verwendeten Begriffe und Definitionen sind (soweit nicht im Einzelfall abweichend definiert) im breitesten Sinne zu verstehen und haben ihre allgemeine Bedeutung, wie sie auf dem Gebiet der vorliegenden Erfindung aus dem allgemeinen Fachwissen bekannt ist.

Die primäre Aufgabe der vorliegenden Erfindung sowie damit verwandte weitere Aufgaben werden gelöst durch ein Verfahren zur Herstellung einer chemischen Zubereitung in einer Verpackung, nebst Verschluss, vorzugsweise nebst Verschluss und Etikett, für eine chemische Zubereitung,
umfassend ein computerimplementiertes Verfahren zur Überwachung eines oder mehrerer Produktionsattribute eines von einem chemischen Produktionsnetzwerk produzierten Ausgangsmaterials, wobei das Verfahren die folgenden Schritte umfasst:
S1a Bereitstellung von Eingangsmaterialdaten betreffend Eingangsmaterialien für die chemische Zubereitung, die
   - mit einem Eingangsmaterialstrom betreffend die chemische Zubereitung oder mehreren Eingangsmaterialströmen betreffend die chemische Zubereitung
      und
   - mit einem oder mehreren Produktionsattributen betreffend die chemische Zubereitung

   verbunden sind, an eine Computerschnittstelle,
   wobei der eine Eingangsmaterialstrom betreffend die chemische Zubereitung oder die mehreren Eingangsmaterialströme betreffend die chemische Zubereitung dem chemischen Produktionsnetzwerk zugeführt werden;
S2a basierend auf dem einen Produktionsattribut das mit dem einen Eingangsmaterialstrom betreffend die chemische Zubereitung verbunden ist oder auf den mehreren Produktionsattributen, die mit den mehreren Eingangsmaterialströmen betreffend die chemische Zubereitung verbunden sind:
   Zuordnen des einen Produktionsattributs betreffend die chemische Zubereitung oder der mehreren Produktionsattribute betreffend die chemische Zubereitung zu mindestens einem Materialkonto, das mit dem jeweiligen Produktionsattribut verbunden ist;
S3a Bereitstellung eines Ausgangsmaterialidentifikators, der mit dem vom chemischen Produktionsnetzwerk produzierten Ausgangsmaterial, vorzugsweise Ausgangsmaterial umfassend die chemische Zubereitung, verbunden ist;
S4a Zuordnen
   - mindestens eines Produktionsattributs aus dem mindestens einen Materialkonto das mit dem jeweiligen Produktionsattribut zugeordneten Produktionsattribut verbunden ist,
   zu einem Ausgangsmaterialidentifikator.

Die primäre Aufgabe der vorliegenden Erfindung sowie damit verwandte weitere Aufgaben werden gelöst durch ein Verfahren zur Herstellung einer chemischen Zubereitung in einer Verpackung, nebst Verschluss, vorzugsweise nebst Verschluss und Etikett, für eine chemische Zubereitung,
umfassend ein computerimplementiertes Verfahren zur Überwachung eines oder mehrerer Produktionsattribute eines von einem chemischen Produktionsnetzwerk produzierten Ausgangsmaterials, wobei das Verfahren die folgenden Schritte umfasst:
S1a Bereitstellung von Eingangsmaterialdaten betreffend Eingangsmaterialien für die chemische Zubereitung, die
   - mit einem Eingangsmaterialstrom betreffend die chemische Zubereitung oder mehreren Eingangsmaterialströmen betreffend die chemische Zubereitung
      und
   - mit einem oder mehreren Produktionsattributen betreffend die chemische Zubereitung

   verbunden sind, an eine Computerschnittstelle,
   wobei der eine Eingangsmaterialstrom betreffend die chemische Zubereitung oder die mehreren Eingangsmaterialströme betreffend die chemische Zubereitung dem chemischen Produktionsnetzwerk zugeführt werden
      und
   wobei mindestens ein Eingangsmaterialstrom betreffend die chemische Zubereitung ein Tensid umfasst, das einen Anteil an ¹⁴C-Atomen umfasst, vorzugsweise dessen hydrophober Molekülteil einen Anteil an ¹⁴C-Atomen umfasst, welcher einem Alter der entsprechenden Kohlenstoffatome von weniger als 300 Jahren seit der Kohlenstoffdioxid-Fixierung entspricht;
   wobei dem einen Eingangsmaterialstrom betreffend die chemische Zubereitung oder den mehreren Eingangsmaterialströmen betreffend die chemische Zubereitung zumindest ein Produktionsattribut zugeordnet ist, das mit dem Anteil an ¹⁴C-Atomen verknüpft ist;
S2a basierend auf dem einen Produktionsattribut das mit dem einen Eingangsmaterialstrom betreffend die chemische Zubereitung verbunden ist oder auf den mehreren Produktionsattributen, die mit den mehreren Eingangsmaterialströmen betreffend die chemische Zubereitung verbunden sind:
   Zuordnen des einen Produktionsattributs betreffend die chemische Zubereitung oder der mehreren Produktionsattribute betreffend die chemische Zubereitung zu mindestens einem Materialkonto, das mit dem jeweiligen Produktionsattribut verbunden ist;
S3a Bereitstellung eines Ausgangsmaterialidentifikators, der mit dem vom chemischen Produktionsnetzwerk produzierten Ausgangsmaterial, vorzugsweise Ausgangsmaterial umfassend die chemische Zubereitung, verbunden ist;
S4a Zuordnen
   - mindestens eines Produktionsattributs aus dem mindestens einen Materialkonto das mit dem jeweiligen Produktionsattribut zugeordneten Produktionsattribut verbunden ist,
   zu einem ersten Ausgangsmaterialidentifikator betreffend die chemische Zubereitung.

In vielen Fällen wird vorzugsweise dem ersten Ausgangsmaterialidentifikator betreffend die chemische Zubereitung die Bezeichnung "Zirkularität der Rezeptur" zugeordnet.

Die Radiokarbonmethode und somit eine zu unzweideutigen Ergebnissen führende Methode zum Bestimmen des Anteils an ¹⁴C-Atomen, welcher einem Alter der entsprechenden Kohlenstoffatome von weniger als 300 Jahren (mit einer Standardabweichung von weniger als 50 Jahren) seit der Kohlenstoffdioxid-Fixierung entspricht, ist dem Fachmann aus seinem allgemeinen Fachwissen bekannt. Methoden zum Kalibrieren entsprechender Messungen sind dem Fachmann ebenfalls bekannt. Ein Anteil von ¹⁴C-Atomen, welcher einem Alter der entsprechenden Kohlenstoffatome von weniger als 300 Jahren (mit einer Standardabweichung von weniger als 50 Jahren) seit der Kohlenstoffdioxid-Fixierung entspricht, ist charakteristisch für Tenside, die aus nachwachsenden Rohstoffen hergestellt sind und schließt aus, dass es sich bei dem untersuchten Tensid um ein Tensid aus rein petrochemischer Quelle handelt.

Das computerimplementierte Verfahren kann von jeder Kombination aus generalisierter Computerhardware und entsprechender Software ausgeführt werden, die, wenn ausgeführt auf der Computerhardware, die Computerhardware dazu nutzt die Funktionen des Verfahrens auszuführen. Das computerimplementierte Verfahren kann aber auch von einer spezialisierten Hardware ausgeführt werden, bei der die Funktionen in der Hardware implementiert sind. Das computerimplementierte Verfahren kann auch mit verteiltem Rechnen ausgeführt werden, wobei eine Vielzahl vom Computern die Funktionen des Verfahrens gemeinsam ausführen. Das Bereitstellen von Daten kann grundsätzlich ein Empfangen von Daten aus anderen Datenquellen sein. Das Bereitstellen kann aber auch ein Zugreifen auf gespeicherte Daten beinhalten.

Die Eingangsmaterialdaten enthalten eine Kennung des Eingangsmaterials/der Eingangsmaterialien und Produktionsattribute, die mit dem Eingangsmaterial/den Eingangsmaterialien verbunden sind. Die Kennung des Eingangsmaterials/der Eingangsmaterialien kann mit der physischen Beschaffenheit und/der Menge des Eingangsmaterials/der Eingangsmaterialien verbunden sein, das/ in das in das chemische Produktionsnetzwerk eintritt/eintreten. Die Materialdaten können bei, vor oder nach Bereitstellung des einen Eingangsmaterials oder der mehreren Eingangsmaterialien an den Eingangspunkten des chemischen Produktionsnetzwerks bereitgestellt werden.

Eingangsmaterialdaten können über ein Kommunikationsnetz an eine Computerschnittstelle des Produktionsbetriebssystems übermittelt werden. Ein Datenlieferant, beispielsweise ein QR-Code-Leser, kann so konfiguriert sein, dass er Materialdaten in Bezug auf ein Eingangsmaterial oder mehrere Eingangsmaterialien und entsprechende Produktionsattribute an eine Computerschnittstelle übermittelt.

Der Begriff "Tensid" beschreibt eine Substanz, deren chemische Struktur durch einen unpolaren (hydrophoben) und einen polaren (hydrophilen) Molekülteil gekennzeichnet ist. Diese amphiphile Molekülstruktur eines Tensids bewirkt eine gewisse Löslichkeit sowohl in hydrophilen als auch hydrophoben Flüssigkeiten. Zudem resultiert aus dieser Struktur die für Tenside charakteristische Oberflächenaktivität. Durch ihre Oberflächenaktivität bewirken Tenside eine Erniedrigung der Oberflächenspannung bzw. Grenzflächenspannung und fördern so die Benetzung von Oberflächen (beispielsweise die Benetzung einer zu reinigenden Oberfläche und/oder die Benetzung von Anschmutzungen durch Wasser), und die Verbesserung der Durchmischbarkeit (Emulgierung) zweier Flüssigkeiten (verglichen mit der Durchmischbarkeit dieser Flüssigkeiten in Abwesenheit des Tensids).

Tenside sind aus dem Stand der Technik bekannt und der Fachmann entnimmt jeweils aus seinem allgemeinen Fachwissen und/oder dem Stand der Technik ob es sich bei einer Substanz um ein Tensid handelt oder nicht.

Dem Fachmann sind insbesondere jeweils eine Vielzahl von Tensiden aus den Tensidklassen der anionischen Tenside, der nichtionischen Tenside, der kationischen Tenside und der amphoteren Tenside bekannt.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet),
wobei das computerimplementierte Verfahren zusätzlich die folgenden Schritte umfasst:
S1b Bereitstellung von Eingangsmaterialdaten betreffend Eingangsmaterialien für die Verpackung, die
   - mit einem Eingangsmaterialstrom betreffend die Verpackung oder mehreren Eingangsmaterialströmen betreffend die Verpackung und
   - mit einem oder mehreren Produktionsattributen betreffend die Verpackung

   verbunden sind, an eine Computerschnittstelle,
   wobei der eine Eingangsmaterialstrom betreffend die Verpackung oder die mehreren Eingangsmaterialströme betreffend die Verpackung dem chemischen Produktionsnetzwerk zugeführt werden
      und
   wobei mindestens ein Eingangsmaterialstrom betreffend die Verpackung ein Post-Consumer Recycled Material (PCR) umfasst;
   wobei dem einen Eingangsmaterialstrom betreffend die Verpackung oder den mehreren Eingangsmaterialströmen betreffend die Verpackung zumindest ein Produktionsattribut zugeordnet ist, das mit dem Anteil an Post-Consumer Recycled Material (PCR) verknüpft ist;
S2b basierend auf dem einen Produktionsattribut, das mit dem einen Eingangsmaterialstrom verbunden ist oder auf den mehreren Produktionsattributen, die mit den mehreren Eingangsmaterialströmen verbunden sind:
   Zuordnen des einen Produktionsattributs betreffend die Verpackung oder der mehreren Produktionsattribute betreffend die Verpackung zu mindestens einem Materialkonto, das mit dem jeweiligen Produktionsattribut verbunden ist;
S3b Bereitstellung eines Ausgangsmaterialidentifikators, der mit dem vom chemischen Produktionsnetzwerk produzierten Ausgangsmaterial verbunden ist;
S4b Zuordnen
   - mindestens eines Produktionsattributs betreffend die Verpackung aus dem mindestens einen Materialkonto, das mit dem jeweiligen Produktionsattribut zugeordneten Produktionsattribut verbunden ist,
   zu einem zweiten Ausgangsmaterialidentifikator betreffend die Verpackung.

In vielen Fällen wird vorzugsweise dem zweiten Ausgangsmaterialidentifikator betreffend die Verpackung die Bezeichnung "Zirkularität der Verpackung" zugeordnet.

Der Begriff "Post-Consumer Recycled Material" sowie die entsprechende Abkürzung "PCR" bezeichnen im Rahmen der vorliegenden Erfindung ein sekundäres Kunststoffmaterial, das aus Kunststoffabfällen (beispielsweise aus Produktverpackungen und/oder Umverpackungen) von Endverbrauchern eines ursprünglichen Kunststoffprodukts (beispielsweise private Haushalte, gewerbliche und industrielle Einrichtungen oder Institute etc.) durch mechanisches Recycling hergestellt wird. Zur Herstellung von Post-Consumer Recycled Material (PCR) werden beispielsweise Kunststoffabfälle aus haushaltsnahen, kommunalen Sammelsystemen erfasst, in Sortieranlagen getrennt/fraktioniert, anschließend aufbereitet (inklusive ggfs. erforderlicher Reinigung) und dann mechanisch zu einem hochwertigen Recyclat-Granulat (Post-Consumer Recycled Material) umgearbeitet, das wie primäres Kunststoffgranulat industriell zu neuen Kunststoffprodukten (Post-Consumer Recycled Material) verarbeitet werden kann. Der Begriff "Post-Consumer Recycled Material" sowie die entsprechende Abkürzung "PCR" bezeichnen im Rahmen der vorliegenden Erfindung auch ein sekundäres Papiermaterial, das aus Papierabfällen (beispielsweise aus alten Zeitungen, aus alten Zeitschriften, aus Produktverpackungen und/oder Umverpackungen) von Endverbrauchern eines ursprünglichen Papierprodukts (beispielsweise private Haushalte, gewerbliche und industrielle Einrichtungen oder Institute etc.) durch Recycling hergestellt wird. Das Recyceln von Papier ist aus dem allgemeinen Fachwissen bekannt.

Unter dem Begriff "mechanisches Recycling" wird dabei (im Einklang mit dem im allgemeinen Fachwissen üblichen Verständnis) eine Verarbeitung von Kunststoffabfällen zu Sekundärrohstoffen oder Produkten verstanden, bei der die chemischen Verbindungen der Kunststoffe nicht aufgespalten werden; insbesondere werden Kettenlängen von Polymeren bei mechanischen Recycling nicht in wesentlichem Ausmaß, vorzugsweise gar nicht, verändert. Das mechanische Recycling von Kunststoffabfällen (vorzugsweise Post-Consumer Material) ist in vielen Fällen kompliziert, da Kunststoffabfälle oft kontaminiert sind und eine Vielzahl von Materialien enthalten, die schwierig voneinander zu trennen sind.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet),
wobei das computerimplementierte Verfahren zusätzlich die folgenden Schritte umfasst:
S1c Bereitstellung von Eingangsmaterialdaten betreffend Eingangsmaterialien für den Verschluss, die
   - mit einem Eingangsmaterialstrom betreffend den Verschluss oder mehreren Eingangsmaterialströmen betreffend den Verschluss und
   - mit einem oder mehreren Produktionsattributen betreffend den Verschluss

   verbunden sind, an eine Computerschnittstelle,
   wobei der eine Eingangsmaterialstrom betreffend den Verschluss oder die mehreren Eingangsmaterialströme betreffend den Verschluss dem chemischen Produktionsnetzwerk zugeführt werden
      und
   wobei mindestens ein Eingangsmaterialstrom betreffend den Verschluss einen Anteil an Post-Consumer Recycled Material (PCR) umfasst;
   wobei dem einen Eingangsmaterialstrom betreffend den Verschluss oder den mehreren Eingangsmaterialströmen betreffend den Verschluss zumindest ein Produktionsattribut zugeordnet ist, das mit dem Anteil an Post-Consumer Recycled Material (PCR) verknüpft ist;
S2c basierend auf dem einen Produktionsattribut, das mit dem einen Eingangsmaterialstrom verbunden ist oder auf den mehreren Produktionsattributen, die mit den mehreren Eingangsmaterialströmen verbunden sind:
   Zuordnen des einen Produktionsattributs betreffend den Verschluss oder der mehreren Produktionsattribute betreffend den Verschluss zu mindestens einem Materialkonto, das mit dem jeweiligen Produktionsattribut verbunden ist;
S3c Bereitstellung eines Ausgangsmaterialidentifikators, der mit dem vom chemischen Produktionsnetzwerk produzierten Ausgangsmaterial verbunden ist;
S4c Zuordnen
   - mindestens eines Produktionsattributs betreffend den Verschluss aus dem mindestens einen Materialkonto, das mit dem jeweiligen Produktionsattribut zugeordneten Produktionsattribut verbunden ist,
   zu einem dritten Ausgangsmaterialidentifikator betreffend den Verschluss.

In vielen Fällen wird vorzugsweise dem dritten Ausgangsmaterialidentifikator betreffend den Verschluss die Bezeichnung "Zirkularität des Verschlusses" zugeordnet.

Bevorzugt ist ein Verfahren zur Herstellung einer chemischen Zubereitung in einer Verpackung, nebst Verschluss und Etikett, für eine chemische Zubereitung (wie vorstehend beschrieben, vorzugsweise wie vorstehend als bevorzugt bezeichnet),
wobei die Verpackung eine Verpackung mit einem daran angebrachten Etikett ist und
wobei das computerimplementierte Verfahren zusätzlich die folgenden Schritte umfasst:
   S1d Bereitstellung von Eingangsmaterialdaten betreffend Eingangsmaterialien für das Etikett, die
      - mit einem Eingangsmaterialstrom betreffend das Etikett oder mehreren Eingangsmaterialströmen betreffend das Etikett und
      - mit einem oder mehreren Produktionsattributen betreffend das Etikett verbunden sind, an eine Computerschnittstelle,

      wobei der eine Eingangsmaterialstrom betreffend das Etikett oder die mehreren Eingangsmaterialströme betreffend das Etikett dem chemischen Produktionsnetzwerk zugeführt werden
         und
      wobei mindestens ein Eingangsmaterialstrom betreffend das Etikett einen Anteil an Post-Consumer Recycled Material (PCR) umfasst;
      wobei dem einen Eingangsmaterialstrom betreffend das Etikett oder den mehreren Eingangsmaterialströmen betreffend das Etikett zumindest ein Produktionsattribut zugeordnet ist, das mit dem Anteil an Post-Consumer Recycled Material (PCR) verknüpft ist;
   S2d basierend auf dem einen Produktionsattribut, das mit dem einen Eingangsmaterialstrom betreffend das Etikett verbunden ist oder auf den mehreren Produktionsattributen, die mit den mehreren Eingangsmaterialströmen betreffend das Etikett verbunden sind:
      Zuordnen des einen Produktionsattributs betreffend das Etikett oder der mehreren Produktionsattribute betreffend das Etikett zu mindestens einem Materialkonto, das mit dem jeweiligen Produktionsattribut verbunden ist;
   S3d Bereitstellung eines Ausgangsmaterialidentifikators, der mit dem vom chemischen Produktionsnetzwerk produzierten Ausgangsmaterial umfassend das Etikett verbunden ist;
   S4d Zuordnen
      - mindestens eines Produktionsattributs betreffend das Etikett aus dem mindestens einen Materialkonto, das mit dem jeweiligen Produktionsattribut zugeordneten Produktionsattribut verbunden ist,
      zu einem vierten Ausgangsmaterialidentifikator betreffend das Etikett.

In vielen Fällen wird vorzugsweise dem vierten Ausgangsmaterialidentifikator betreffend den Verschluss die Bezeichnung "Zirkularität des Etiketts" zugeordnet.

Chemische Produktionsnetzwerke sind groß angelegte Produktionsnetzwerke, die tausende von chemischen Produkten durch chemische Umwandlung produzieren. Um negative Umwelteinflüsse der Verfahren und der Produkte zu reduzieren, unterliegen chemischen Produktionsnetze dynamischen Veränderungen. Diese betreffen insbesondere die für solche chemischen Produktionsnetze verwendeten Einsatzstoffe. Einsatzstoffe unterschiedlicher Art müssen dem chemischen Produktionsnetzwerk in kontrollierter Weise zugeführt werden, wobei gleichzeitig die negativen Umwelteinflüsse minimiert werden sollen.

Da es sich bei den chemischen Produktionsnetzen um integrierte chemische Produktionsnetze handelt, in denen verschiedene Produktionsketten miteinander verbunden sind und sie mehrere Arten von Produktionsprozessen zur Herstellung mehrerer Ausgangsmaterialien aus mehreren Eingangsmaterialien umfassen, ist die Überwachung der Auswirkungen der Einsatzstoffe auf die Umweltleistung der vom chemischen Produktionsnetzwerk produzierten Ausgangsstoffe des chemischen Produktionsnetzes eine große Herausforderung.

Eine zuverlässige Überwachung der Produktionseigenschaften der Ausgangsstoffe aus chemischen Produktionsnetzwerken ist daher angesichts der komplexen Zusammensetzung und zunehmenden Anzahl der verwendeten Einsatzstoffe von entscheidender Bedeutung. Mit mehreren verschiedenen Arten von Einsatzstoffen, die in solche chemischen Produktionsnetze einfließen, wird die zuverlässige Überwachung der Stoffströme innerhalb des chemischen Produktionsnetzwerks eine zunehmende Herausforderung.

Insbesondere durch die Zuordnung und Zuweisung von Produktionseigenschaften, die mit den Einsatzstoffen verknüpft sind, zu Materialkonten gelingt die Zuordnung von Produktionseigenschaften von Einsatzmaterialien (beim Eintritt in das chemische Produktionsnetzwerk) zu den Ausgangsmaterialien (beim Austritt aus dem chemischen Produktionsnetzwerk bzw. beim Austritt aus Abschnitten des chemischen Produktionsnetzwerks).

Auf diese Weise kann die Überwachung losgelöst werden von den komplexen Stoffströmen, die in chemischen Produktionsnetzwerken aufgrund der chemischen Verarbeitung und/oder fehlenden technologischen Möglichkeiten nicht mit der physikalischen Spezifität der Eingangsmaterialien verfolgt werden können.

Eine solche Verfolgung wird immer wichtiger für zirkuläre Wertschöpfungsketten, in denen recyceltes Material aus verschiedenen Quellen (insbesondere aus verschiedenen nachhaltigen und/oder pflanzenbasierten Quellen), Abfallströmen, Endprodukten oder Materialarten in chemische Produktionsnetzwerke eingespeist werden. Die Verwendung von Materialkonten für Produktionsmerkmale ermöglicht eine zuverlässige und einfache Überwachung der Materialflüsse in chemischen Produktionsnetzwerken.

Der Begriff "Eingangsmaterialstrom" bzw. der Begriff "Eingangsmaterialströme" umfasst jedes Eingangsmaterial, das über die Systemgrenze in das chemische Produktionsnetzwerk gelangt.

Der Begriff "Ausgangsmaterial" umfasst jedes Ausgangsmaterial, das die Systemgrenze des chemischen Produktionsnetzwerks verlässt.

Das chemische Produktionsnetzwerk kann mehrere Produktionsschritte oder -prozesse umfassen. Die Systemgrenze des chemischen Produktionsnetzes kann durch die Produktionsschritte oder-prozesse definiert werden. Die Systemgrenze kann durch den Standort oder die Kontrolle über Produktionsschritte oder -prozesse definiert werden. Die Systemgrenze kann durch Produktionsschritte oder Produktionsprozesse definiert sein, die von einer oder mehreren Stellen gemeinsam kontrolliert werden. Die Systemgrenze kann definiert sein durch eine Wertschöpfungskette mit gestaffelten Produktionsprozessen zu einem Endprodukt, die von mehreren Unternehmen getrennt kontrolliert werden können.

Netzwerk kann einen, zwei oder mehrere Schritte der Abfallsammlung, einen, zwei oder mehrere Sortierschritte, einen, zwei oder mehrere Recyclingschritte (insbesondere mechanisches Recycling von Kunststoffen), einen, zwei oder mehrere Produktionsschritte (insbesondere Herstellung von Kunststoffen, Herstellung von Tensiden, Herstellung von Waschund Reinigungshilfsmitteln, Herstellung von Verpackungen, Herstellung von Verschlüssen für Verpackungen etc.), einen, zwei oder mehrere Trennungsschritte zur Trennung der Ausgänge eines Prozesses sowie weitere Verarbeitungsschritte zur Umwandlung dieser Ausgänge in ein Ausgangsmaterial (vorzugsweise ein Ausgangsmaterial das die Systemgrenze des chemischen Produktionsnetzes verlässt) umfassen.

Der Eingangsmaterialstrom bzw. die Eingangsmaterialströme können mit Materialdaten verknüpft werden, umfassend
- zumindest einen Eingangsmaterialidentifikator, der mit dem jeweiligen Eingangsmaterial oder Eingangsmaterialstrom verknüpft ist,
- mindestens ein Produktionsattribut, das mit dem jeweiligen Eingangsmaterial oder Eingangsmaterialstrom verknüpft ist
   und/oder
- mit einer Menge insbesondere einer Menge an Eingangsmaterial, die über die Systemgrenze des chemischen Produktionsnetzes dem chemischen Produktionsnetzwerk zugeführt wird.

Die Kennung des Eingangsmaterials kann verknüpft sein mit der physischen Einheit des Eingangsmaterials bzw. des Eingangsmaterialstroms. Die Kennung des Eingangsmaterialidentifikators kann eindeutig mit der physischen Einheit des Eingangsmaterials oder des Eingangsmaterialstroms verknüpft sein. Auf diese Weise kann die virtuelle oder digitale Kennung des Eingangsmaterials eindeutig mit dem physischen Eingangsmaterial verknüpft werden. Eine solche Verknüpfung kann eine physische oder virtuelle Verknüpfung von Identifikatoren umfassen, die eindeutig mit dem physischen Eingangsmaterial verbunden sind. Bei der physischen Verknüpfung kann ein Tag oder Code physisch mit dem Material verbunden werden, z. B. durch Aufdruck eines QR-Codes auf der Verpackung. Für virtuellen Verknüpfung können verschiedene Identifikatoren mit dem physischen Material verknüpft werden. Beispielsweise kann eine Bestellnummer, eine Chargennummer, eine LOT-Nummer oder eine Kombination davon verknüpft werden.

Die ein oder mehreren Produktionsattribute können sich auf Eigenschaften des Ausgangsmaterials beziehen, die durch die Produktion des Ausgangsmaterials unter Verwendung von Eingangsmaterialströmen und/oder Produktionsprozessen entstehen. Das eine Produktionsattribut oder die mehreren Produktionsattribute können sich auf Eigenschaften des Eingangsmaterialstroms bzw. der Eingangsmaterialströme und/oder der Produktionsprozesse (einschließlich Recyclingprozesse) beziehen, die in der Produktionskette des Ausgangsmaterials eingesetzt werden.

Das Produktionsattribut kann einen digitalen Wert enthalten, der
- dem Eingangsmaterial und/oder dem Eingangsmaterialstrom bzw. den Eingangsmaterialströmen
   und/oder
- dem Ausgangsmaterial und/oder einem Ausgangsmaterialstrom bzw. Ausgangsmaterialströmen
   zugeordnet ist.

Das Produktionsattribut kann digital eine Eigenschaft oder mehrere Eigenschaften des Eingangsmaterialstroms bzw. der Eingangsmaterialströme oder des Ausgangsmaterialstroms bzw. der Ausgangsmaterialströme digital angeben.

Die Produktionsattribute können sich auf einen Materialtyp und/oder einen Prozesstyp, insbesondere auf einen Recyclingmaterialtyp und/oder einen Recyclingprozesstyp beziehen. Die Art des Materials, insbesondere Recyclingmaterials, kann sich auf die Eigenschaften des recycelten oder zu recycelnden Eingangsmaterials beziehen.

Der Recyclingverfahrenstyp kann sich auf die Eigenschaften des Verfahrens beziehen, mit dem das Einsatzmaterial recycelt wurden oder recycelt werden soll.

Die Produktionsattribute können sich auf ein Umweltattribut bzw. mehrere Umweltattribute beziehen. Das Umweltattribut kann einen digitalen Wert enthalten, der mit dem Eingangsmaterial oder dem Ausgangsmaterial verbunden ist. Das Umweltattribut (bzw. die Umweltattribute) kann die Umweltauswirkungen des Eingangsmaterials oder des Ausgangsmaterials digital spezifizieren.

Das Umweltattribut (bzw. die Umweltattribute) kann sich auf einen erneuerbaren, einen biobasierten und/oder einen recycelten Inhalt, beispielsweise des Eingangsmaterials und/oder Ausgangsmaterials, beziehen. Das Umweltattribut (bzw. die Umweltattribute) kann einen qualitativen Datenpunkt enthalten, der sich auf die Art der Auswirkung bezieht, beispielsweise im Hinblick auf das Einsatzmaterial oder das Ausgangsmaterial.

Das Umweltattribut (bzw. die Umweltattribute) kann einen Typ angeben, wie beispielsweise recycelt, erneuerbar und/oder biobasiert. Der qualitative Datenpunkt (angegebener Typ) kann umgewandelt werden in ein quantitatives Maß wie beispielsweise Bilanzierungseinheiten. Das Umweltattribut (bzw. die Umweltattribute) kann einen quantitativen Datenpunkt enthalten, der sich auf die Art der Auswirkung bezieht, beispielsweise in Bezug auf das Ausgangsmaterial. Das Umweltattribut (bzw. die Umweltattribute) kann den rezyklierten, erneuerbaren und/oder biobasierten Anteil angeben.

Das Umweltattribut (bzw. die Umweltattribute) kann sich auf eine beliebige Eigenschaft in Bezug auf die Umweltauswirkungen beziehen. Eine solche Eigenschaft kann eine Eigenschaft des Eingangsmaterials bzw. der Eingangsmaterialien oder des Ausgangsmaterials bzw. der Ausgangsmaterialien sein.

Das Umweltattribut (bzw. die Umweltattribute) kann sich auf die Umweltverträglichkeit
- des Eingangsmaterials bzw. der Eingangsmaterialien,
- des chemischen Produktionsnetzes
   und/oder
- des Ausgangsmaterials bzw. der Ausgangsmaterialien,
   beziehen.

Das Umweltattribut (bzw. die Umweltattribute) kann abgeleitet werden aus den Eigenschaften
- des Eingangsmaterials bzw. der Eingangsmaterialien,
- des chemischen Produktionsnetzes
   und/oder
- des Ausgangsmaterials bzw. der Ausgangsmaterialien.

Das Umweltattribut (bzw. die Umweltattribute) kann mit den Umweltauswirkungen eines oder mehrerer Materialien in einem beliebigen Stadium ihres Lebenszyklus verknüpft sein. Die Stadien des Material- oder Produktlebenszyklus können die Stadien der Bereitstellung von Rohstoffen, der Herstellung von Produkten (wie Zwischenprodukte oder Endprodukte), der Nutzung von Produkten, der Behandlung von Altprodukten, Recycling von Altprodukten, Entsorgung von Altprodukten, Wiederverwendung von Komponenten von Altprodukten oder beliebige Untergruppe der genannten Stadien umfassen.

Das Umweltattribut (bzw. die Umweltattribute) kann durch jede Tätigkeit einer oder mehrerer Stellen verfolgt werden, die an einer beliebigen Phase des Lebenszyklus eines oder mehrerer Materialien oder Produkte beteiligt sind. Umweltattribute im Zusammenhang mit einer Tätigkeit einer oder mehrerer Stellen, die an einem beliebigen Stadium des Lebenszyklus eines Materials oder mehrerer Materialien und/oder eines Produkts oder mehrerer Produkte beteiligt sind, können je nach den Erfordernissen des Einzelfalls akkumuliert oder aggregiert werden.

Das Umweltattribut (bzw. die Umweltattribute) kann ein oder mehrere Merkmale umfassen, die auf die Umweltauswirkungen des Eingangsmaterials bzw. der Eingangsmmaterialien, des Ausgangsmaterials bzw. der Ausgangsmaterialien und/oder von Zwischenprodukten zurückzuführen sind.

Das Umweltattribut (bzw. die Umweltattribute) kann ökologische Kriterien spezifizieren oder quantifizieren im Zusammenhang mit Umweltauswirkungen von Einsatzmaterial, Ausgangsmaterial und/oder Zwischenprodukt.

In vielen Fällen spezifiziert das Produktionsattribut, bevorzugt Umweltattribut, einen Gesamtanteil von tensidischen und nicht-tensidischen ¹⁴C-Atomen, welcher einem Alter der entsprechenden Kohlenstoffatome von weniger als 300 Jahren seit der Kohlenstoffdioxid-Fixierung entspricht, in einem Eingangsmaterialstrom (vorzugsweise Eingangsmaterialstrom betreffend die chemische Zubereitung) und/oder in einem Ausgangsmaterialstrom (vorzugsweise Ausgangsmaterialstrom betreffend die chemische Zubereitung).

In vielen Fällen spezifiziert das Produktionsattribut, bevorzugt Umweltattribut, einen Anteil eines Eingangsmaterialstroms (vorzugsweise Eingangsmaterialstrom betreffend die chemische Zubereitung) der hergestellt ist aus einer Pflanze; vorzugsweise hergestellt ist aus einer Pflanze, die ausgewählt ist aus der Gruppe bestehend aus:
- Nadelbäume (insbesondere Douglasie, Eibe, Fichte, Kiefer, Lärche, Nootka-Scheinzypresse, Riesen-Lebensbaum, Tanne und Weymouth-Kiefer), Laubbäume (insbesondere Ahorn, Balsa, Birke, Buche, Eiche, Erle, Esche, Espe, Kirsche, Linde, Pappel, Platane, Robinie, Ulme (Rüster), Walnuss und Weißbuche), Hanf, Haselnuss, Distel, Erdnuss, Erdmandel, Gräser, Getreide (insbesondere Weizen), Kartoffel, Jojoba, Iberischer Drachenkopf, Kokospalme, Leindotter, Flachs, Mais, Mandel, Mohn, Nachtkerze, Olive, Ölpalme, Ölrettich, Ölrauke, Pekannuss, Pistazie, Raps, Rizinus, Rübsen, Saflor, Schwarzkümmel, Sesam, Sonnenblume, Hülsenfrüchtler, insbesondere Soja, Walnuss, Weintraube, Zuckerrübe, Zuckerrohr und Kombinationen daraus;

In vielen Fällen werden vorzugsweise im Verfahren Eingangsmaterialströme (vorzugsweise Eingangsmaterialstrom betreffend die chemische Zubereitung) ausgeschlossen bzw. deren Einsatz minimiert, bei denen Anteile der Eingangsmaterialströme aus einer Pflanzenart stammen, die nicht ausgewählt ist aus der vorstehend definierten Gruppe.

In vielen Fällen spezifiziert das Produktionsattribut, bevorzugt Umweltattribut, einen Massenanteil aerob leicht abbaubarer Substanzen, in einem Eingangsmaterialstrom (vorzugsweise Eingangsmaterialstrom betreffend die chemische Zubereitung) und/oder in einem Ausgangsmaterialstrom (vorzugsweise Ausgangsmaterialstrom betreffend die chemische Zubereitung).

Der Begriff "aerob leicht abbaubare Substanz" bezeichnet im Rahmen der vorliegenden Erfindung organische Materialien, die gemäß OECD 301, Adopted 17.07.92 ( OECD GUIDELINE FOR TESTING OF CHEMICALS, Adopted by the Council on 17th July 1992, Ready Biodegradability) als "readily biodegradable" eingestuft werden. Substanzen mit dieser Eigenschaft sind im Rahmen der Abwasseraufbereitung in kommunalen Kläranlagen leicht zu entfernen. Der Fachmann führt den Nachweis dass eine Substanz eine "aerob leicht abbaubare Substanz" ist experimentell ohne unzumutbaren Aufwand gemäß der in der vorstehend spezifizierten OECD 301 definierten Vorgehensweise durch.

In vielen Fällen spezifiziert das Produktionsattribut, bevorzugt Umweltattribut, einen Massenanteil anaerob abbaubarer Substanzen, in einem Eingangsmaterialstrom (vorzugsweise Eingangsmaterialstrom betreffend die chemische Zubereitung) und/oder in einem Ausgangsmaterialstrom (vorzugsweise Ausgangsmaterialstrom betreffend die chemische Zubereitung).

Der Begriff "anaerob abbaubare Substanz" bezeichnet im Rahmen der vorliegenden Erfindung organische Materialien, die in Untersuchungen gemäß OECD 311, Adopted 23. Marach 2006 (OECD GUIDELINE FOR TESTING OF CHEMICALS, Anaerobic Biodegradability ofOrganic Compounds in Digested Sludge: By Measurement of Gas Production) eine vollständige anerobe Biodegradation (complete anaerobic biodegradation) zeigen. Substanzen mit dieser Eigenschaft sind im Rahmen der Abwasseraufbereitung in kommunalen Kläranlagen leicht zu entfernen. Der Fachmann führt den Nachweis, dass eine Substanz eine "anaerob abbaubare Substanz" ist, experimentell ohne unzumutbaren Aufwand gemäß der in der vorstehend spezifizierten OECD 311 definierten Vorgehensweise durch.

In vielen Fällen spezifiziert das Produktionsattribut, bevorzugt Umweltattribut, einen Massenanteil biologisch hochwertig kreislauffähiger Materialien, in einem Eingangsmaterialstrom (vorzugsweise Eingangsmaterialstrom betreffend die chemische Zusammensetzung) und/oder in einem Ausgangsmaterialstrom (vorzugsweise Ausgangsmaterialstrom betreffend die chemische Zusammensetzung).

Der Begriff "biologisch hochwertig kreislauffähiges Material" bzw. "biologisch hochwertig kreislauffähige Inhaltsstoffe" bezeichnet organische Materialien, die gemäß der vorangehenden Definitionen sowohl "aerob leicht abbaubare Substanz" als auch zudem "anaerob abbaubare Substanz" sind.

In vielen Fällen spezifiziert das Produktionsattribut, bevorzugt Umweltattribut, einen Massenanteil mechanisch rezyklierbarer Materialien in einem Eingangsmaterialstrom (vorzugsweise Eingangsmaterialstrom betreffend die Verpackung) und/oder in einem Ausgangsmaterialstrom (vorzugsweise Ausgangsmaterialstrom betreffend die Verpackung).

Der Begriff "mechanisch rezyklierbare Materialien" bezeichnet Materialien aus Kunststoff, aus denen durch ausschließlich mechanisches Recycling ein sekundäres Kunststoffmaterial zur Herstellung technisch voll funktionsfähiger Neuprodukte hergestellt werden kann. Das aus dem mechanischen Recycling mechanisch rezyklierbarer Materialien resultierende sekundäre Kunststoffmaterial kann dabei eine chemisch und optisch gegenüber einem entsprechenden Primärmaterial veränderte Qualität aufweisen (beispielswiese durch in das Kunststoffmaterial eingebrachte Farbpigmente). Im Stand der Technik führt ein mechanisches Recycling farbiger Kunststoffmaterialien (mit Ausnahme weiß eingefärbter Monokunststoff-Materialien) regelmäßig zu einem Verlust zumindest an optischer Qualität der Sekundärmaterialien ("Downcycling"), da ein farbreines mechanisches Recycling von mechanisch rezyklierbaren Materialien technisch und wirtschaftlich regelmäßig nicht industriell effizient umsetzbar ist.

In vielen Fällen spezifiziert das Produktionsattribut, bevorzugt Umweltattribut, einen Massenanteil mechanisch upcyclingfähiger Materialien, in einem Eingangsmaterialstrom (vorzugsweise Eingangsmaterialstrom betreffend die Verpackung) und/oder in einem Ausgangsmaterialstrom (vorzugsweise Ausgangsmaterialstrom betreffend die Verpackung).

Der Begriff "mechanisch upcyclingfähige Materialien" bezeichnet im Rahmen der vorliegenden Erfindung Materialien aus Kunststoff, aus denen durch ausschließlich mechanisches Recycling ein sekundäres Kunststoffmaterial zur Herstellung technisch voll funktionsfähiger sekundärer Neuprodukte hergestellt werden kann, ohne dass die sekundären Neuprodukte technisch, optisch und/oder (bevorzugt "und") chemisch gegenüber einem entsprechenden Primärmaterial eine veränderte Qualität aufweisen. Im Stand der Technik sind nur solche Materialien als "mechanisch upcyclingfähige Materialien" bekannt, die keine Farbstoffe oder keine Farbstoffe außer weißen Farbstoffen enthalten.

"Mechanisch upcyclingfähige Materialien" werden auf dem Gebiet der vorliegenden Erfindung auch als "mechanisch qualitätserhaltend recycelbare Materialen" bezeichnet.

"Mechanisch upcyclingfähige Materialien" sind "mechanisch rezyklierbare Materialien".

In vielen Fällen spezifiziert das Produktionsattribut, bevorzugt Umweltattribut, einen kombinierten Massenanteil ungefärbter und weißer Kunststoffkomponenten, in einem Eingangsmaterialstrom (vorzugsweise Eingangsmaterialstrom betreffend die Verpackung) und/oder in einem Ausgangsmaterialstrom (vorzugsweise Ausgangsmaterialstrom betreffend die Verpackung).

In vielen Fällen spezifiziert das Produktionsattribut, bevorzugt Umweltattribut, einen Massenanteil biologisch hochwertig kreislauffähiger Materialien, in einem Eingangsmaterialstrom (vorzugsweise Eingangsmaterialstrom betreffend den Verschluss) und/oder in einem Ausgangsmaterialstrom (vorzugsweise Ausgangsmaterialstrom betreffend den Verschluss).

In vielen Fällen spezifiziert das Produktionsattribut, bevorzugt Umweltattribut, einen Massenanteil mechanisch rezyklierbarer Materialien, in einem Eingangsmaterialstrom (vorzugsweise Eingangsmaterialstrom betreffend den Verschluss) und/oder in einem Ausgangsmaterialstrom (vorzugsweise Ausgangsmaterialstrom betreffend den Verschluss).

In vielen Fällen spezifiziert das Produktionsattribut, bevorzugt Umweltattribut, einen Massenanteil mechanisch upcyclingfähiger Materialien, in einem Eingangsmaterialstrom (vorzugsweise Eingangsmaterialstrom betreffend den Verschluss) und/oder in einem Ausgangsmaterialstrom (vorzugsweise Ausgangsmaterialstrom betreffend den Verschluss).

In vielen Fällen spezifiziert das Produktionsattribut, bevorzugt Umweltattribut, einen kombinierten Massenanteil ungefärbter und weißer Kunststoffkomponenten, in einem Eingangsmaterialstrom (vorzugsweise Eingangsmaterialstrom betreffend den Verschluss) und/oder in einem Ausgangsmaterialstrom (vorzugsweise Ausgangsmaterialstrom betreffend den Verschluss).

Technische Merkmal können die Produktleistung spezifizieren oder quantifizieren, die zumindest indirekt mit den Umweltauswirkungen verbunden sind.

Das chemische Produktionsnetzwerk kann mehrere Produktionsprozesse oder Produktionsschritte pro Produktionskette umfassen. Die Produktionsprozesse oder Prozessschritte, die in das chemische Produktionsnetzwerk eingehen, können durch die physische Systemgrenze des chemischen Produktionsnetzes definiert werden.

Die Eingangspunkte des chemischen Produktionsnetzwerks sind in vielen Fällen durch den Eingang von Eingangsmaterialien in das chemische Produktionsnetzwerk gekennzeichnet. Die in das chemische Produktionsnetzwerk eintretenden Eingangsmaterialien werden in vielen Fällen zur Herstellung eines oder mehrerer Ausgangsmaterialien verwendet. Die Ausgangsmaterialien können die physikalische Systemgrenze des chemischen Produktionsnetzes verlassen; dies ist regelmäßig bevorzugt.

Die Austrittspunkte des chemischen Produktionsnetzes werden in vielen Fällen durch den Austritt von Ausgangsmaterialien gekennzeichnet.

Das chemische Produktionsnetzwerk kann einen oder mehrere Eingangspunkte umfassen, an denen Eingangsmaterialströme dem chemischen Produktionsnetzwerk zugeführt werden. Eingangsmaterialströme können fossilen Kohlenstoff, nicht-fossilen Kohlenstoff oder beides umfassen. Bevorzugt umfassen die Eingangsmaterialströme weniger als 50 Gew.-% fossilen Kohlenstoff, besonders bevorzugt weniger als 30 Gew.-% ganz besonders bevorzugt weniger als 10 Gew.-% fossiles Material bezogen auf den Gesamtmassefluss des jeweiligen Eingangsmaterialstroms. Fossilen Kohlenstoff identifiziert der Fachmann anhand der ¹⁴C-Methode (Radiokarbonmethode).

Das Materialkonto kann sich auf eine digitale Speicherstruktur beziehen, in der Daten in Bezug auf Produktionseigenschaften gespeichert werden. Das Konto kann mit einem oder mehreren Merkmalen verknüpft sein, die eine Bilanzierung von Produktionsattributen ermöglichen. Das Konto kann mit Merkmalen verknüpft sein, die den dem Konto zugeordneten Produktions- oder Bilanzierungseinheiten zugeordnet sind. Das Konto kann mit einem oder mehreren Merkmalen verknüpft werden, die die Produktionskette identifizieren, mit der das Konto verknüpft ist. Das Konto kann mit einem Merkmal oder mehreren Merkmalen verknüpft sein, die das Eingangsmaterial oder das Ausgangsmaterial identifizieren, mit dem das Konto verbunden ist. Das Konto kann Teil eines digitalen Materialbilanzierungssystems mit mehreren Materialkonten sein.

Das Konto kann Produktionsattribute für Transaktionen enthalten. Produktionsattribute können dem Materialkonto zugewiesen, hinzugefügt, gelöscht, entnommen, und/oder abgezogen werden.

Das Materialkonto kann mit einem oder mehreren Produktionsattributen verbunden sein, wie z. B. recycelt, biobasiert, erneuerbar oder Kombinationen davon.

Das Materialkonto kann mit einem Zuordnungsschema verknüpft sein, wie beispielsweise getrennte Zuordnung, nicht getrennte Zuordnung wie Buch und Forderung (Book and Claim), Massenbilanz mit freier Zuteilung, Massenbilanz ohne freie Zuteilung oder Kombinationen davon.

Das Ausgangsmaterial kann von dem chemischen Produktionsnetzwerk produziert werden, dem die Eingangsmaterialströme, die mit einem oder mehreren Produktionsattributen verbunden sind, bereitgestellt wurden.

Das Ausgangsmaterial kann von einer Produktionskette des chemischen Produktionsnetzes produziert werden, dem mit einem oder mehreren Produktionsnetzen hergestellte und mit ein oder mehreren Produktionsattributen verknüpfte Eingangsmaterialströme zugeführt werden.

Das Ausgabematerial kann aus dem mit einem oder mehreren Produktionsattributen verknüpften Eingangsmaterialstrom bzw. aus den mit einem oder mehreren Produktionsattributen verknüpften Eingangsmaterialströmen produziert werden.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei das computerimplementierte Verfahren zusätzlich den folgenden Schritt umfasst:
S5 Miteinanderverrechnen von zwei, drei oder sämtlichen Ausgangsmaterialindikatoren, ausgewählt aus der Gruppe bestehend aus:
   - erster Ausgangsmaterialidentifikator betreffend die chemische Zubereitung, resultierend in Schritt S4a;
      und
   - zweiter Ausgangsmaterialidentifikator betreffend die Verpackung, resultierend in Schritt S4b;
      und
   - dritter Ausgangsmaterialidentifikator betreffend den Verschluss, resultierend in Schritt S4c;
      und
   - vierter Ausgangsmaterialidentifikator betreffend das Etikett, resultierend in Schritt S4d;
   so dass ein Ausgangsmaterialidentifikator betreffend ein Konsumprodukt resultiert.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei das computerimplementierte Verfahren zusätzlich den folgenden Schritt umfasst:
S5 Miteinanderverrechnen:
   - eines ersten Ausgangsmaterialidentifikators betreffend die chemische Zubereitung, resultierend in Schritt S4a;
      und
   - eines zweiten Ausgangsmaterialidentifikators betreffend die Verpackung, resultierend in Schritt S4b;
      und
   - eines dritten Ausgangsmaterialidentifikators betreffend den Verschluss, resultierend in Schritt S4c;
   so dass ein Ausgangsmaterialidentifikator betreffend ein Konsumprodukt resultiert.

In vielen Fällen wird vorzugsweise dem Ausgangsmaterialidentifikator betreffend die Gesamtheit der chemischen Zubereitung und der Verpackung nebst Verschluss, vorzugsweise nebst Verschluss und Etikett, für eine chemische Zubereitung die Bezeichnung "Gesamtzirkularität" bzw. "Wert gemäß Zirkularimeter" zugeordnet.

Bevorzugt ist ein Verfahren zur Herstellung einer chemischen Zubereitung in einer Verpackung, nebst Verschluss und Etikett, für eine chemische Zubereitung (wie vorstehend beschrieben, vorzugsweise wie vorstehend als bevorzugt bezeichnet), wobei das computerimplementierte Verfahren zusätzlich den folgenden Schritt umfasst:
S5 Miteinanderverrechnen:
   - eines ersten Ausgangsmaterialidentifikators betreffend die chemische Zubereitung, resultierend in Schritt S4a;
      und
   - eines zweiten Ausgangsmaterialidentifikators betreffend die Verpackung, resultierend in Schritt S4b;
      und
   - eines dritten Ausgangsmaterialidentifikators betreffend den Verschluss, resultierend in Schritt S4c;
      und
   - eines vierten Ausgangsmaterialidentifikators betreffend das Etikett, resultierend in Schritt S4d;
   so dass ein Ausgangsmaterialidentifikator betreffend ein Konsumprodukt resultiert.

In vielen Fällen wird vorzugsweise dem Ausgangsmaterialidentifikator betreffend die Gesamtheit der chemischen Zubereitung und der Verpackung, nebst Verschluss und Etikett, für eine chemische Zubereitung die Bezeichnung "Gesamtzirkularität" bzw. "Wert gemäß Zirkularimeter" zugeordnet.

Aspekt A1: Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet),
umfassend ein computerimplementiertes Verfahren zur Überwachung eines oder mehrerer Produktionsattribute eines von einem chemischen Produktionsnetzwerk produzierten Ausgangsmaterials, wobei das Verfahren, vorzugsweise das computerimplementierte Verfahren, die folgenden Schritte umfasst:
S1a Bereitstellung von Eingangsmaterialdaten betreffend Eingangsmaterialien für die chemische Zubereitung, die
   - mit einem Eingangsmaterialstrom betreffend die chemische Zubereitung oder mehreren Eingangsmaterialströmen betreffend die chemische Zubereitung
      und
   - mit einem oder mehreren Produktionsattributen betreffend die chemische Zubereitung

   verbunden sind, an eine Computerschnittstelle,
   wobei der eine Eingangsmaterialstrom betreffend die chemische Zubereitung oder die mehreren Eingangsmaterialströme betreffend die chemische Zubereitung dem chemischen Produktionsnetzwerk zugeführt werden;
S2a basierend auf dem einen Produktionsattribut das mit dem einen Eingangsmaterialstrom betreffend die chemische Zubereitung verbunden ist oder auf den mehreren Produktionsattributen, die mit den mehreren Eingangsmaterialströmen betreffend die chemische Zubereitung verbunden sind:
   Zuordnen des einen Produktionsattributs betreffend die chemische Zubereitung oder der mehreren Produktionsattribute betreffend die chemische Zubereitung zu mindestens einem Materialkonto, das mit dem jeweiligen Produktionsattribut verbunden ist;
S3a Bereitstellung eines Ausgangsmaterialidentifikators, der mit dem vom chemischen Produktionsnetzwerk produzierten Ausgangsmaterial verbunden ist;
S4a Zuordnen
   - mindestens eines Produktionsattributs aus dem mindestens einen Materialkonto das mit dem jeweiligen Produktionsattribut zugeordneten Produktionsattribut verbunden ist,
   zu einem Ausgangsmaterialidentifikator;
wobei das Verfahren zur Herstellung zumindest den folgenden Schritt umfasst:
C1 Bereitstellen einer Rezeptur, vorzugsweise einer Rezeptur umfassend zumindest ein, zwei drei oder mehr Tenside, eines Waschmittels und/oder eines Reinigungsmittels und/oder einer kosmetischen Zubereitung,
   und wobei das Verfahren zusätzlich zumindest einen, bevorzugt zumindest zwei, besonders bevorzugt zumindest drei, ganz besonders bevorzugt sämtliche der folgenden Schritte umfasst:
   C2 Herstellen oder Bereitstellen der Inhaltsstoffe, vorzugsweise in jeweils getrennten Gefäßen, gemäß der in Schritt C1 bereitgestellten Rezeptur
      und
      Bestimmung des Massenanteils an regenerativem Kohlenstoff bezogen auf die Gesamtmasse sämtlicher gemäß der in Schritt C1 bereitgestellten Rezeptur vorhandenen kohlenstoffhaltigen Inhaltstoffe;
         und
      Bereitstellen zumindest eines Produktionsattributs, betreffend den regenerativen Kohlenstoffanteil der bereitgestellten Inhaltstoffe;
      und/oder
   C3 Herstellen oder Bereitstellen der Inhaltsstoffe, vorzugsweise in jeweils getrennten Gefäßen, gemäß der in Schritt C1 bereitgestellten Rezeptur
      und
      Bestimmung des Massenanteils an regenerativem Kohlenstoff in sämtlichen gemäß der in Schritt C1 bereitgestellten Rezeptur vorhandenen Tensiden, bezogen auf die Gesamtmasse aller gemäß der in Schritt C1 bereitgestellten Rezeptur vorhandenen Tenside;
         und
      Bereitstellen zumindest eines Produktionsattributs, betreffend den regenerativen Kohlenstoffanteil der bereitgestellten Inhaltstoffe;
      und/oder
   C4 Herstellen oder Bereitstellen der Inhaltsstoffe, vorzugsweise in jeweils getrennten Gefäßen, gemäß der in Schritt C1 bereitgestellten Rezeptur, wobei das Herstellen oder Bereitstellen der Inhaltsstoffe ein Herstellen von Pflanzenöl umfasst, vorzugsweise aus einer Pflanze der Gruppe bestehend aus:
      - Hanf, Haselnuss, Distel, Erdnuss, Erdmandel, Jojoba, Iberischer Drachenkopf, Kokospalme, Leindotter, Flachs, Mandel, Mohn, Nachtkerze, Olive, Ölpalme, Ölrettich, Ölrauke, Pekannuss, Pistazie, Raps, Rizinus, Rübsen, Saflor, Schwarzkümmel, Sesam, Sonnenblume, Soja, Walnuss, Weintraube und Kombinationen daraus;

      sowie das Herstellen von Tensidmolekülen aus diesem Pflanzenöl;
         und
      Bestimmung des Massenanteils der Tenside, die aus Pflanzenöl hergestellt wurden, vorzugsweise den Pflanzen gemäß der obigen Gruppe hergestellt wurden, bezogen auf die Gesamtmasse der gemäß der in Schritt C1 bereitgestellten Rezeptur vorhandenen Tenside;
         und
      Bereitstellen zumindest eines Produktionsattributs, betreffend den Massenanteils der Tenside, die aus den Pflanzen gemäß der obigen Gruppe hergestellt wurden;
      und/oder
   C5 Herstellen oder Bereitstellen der Inhaltsstoffe, vorzugsweise in jeweils getrennten Gefäßen, gemäß der in Schritt C1 bereitgestellten Rezeptur
      und
      Bestimmung des Massenanteils an Inhaltsstoffen die gemäß OECD 301 als aerob leicht abbaubar (Ready Biodegradable) bezeichnet werden, bezogen auf die Gesamtmasse der gemäß der in Schritt C1 bereitgestellten Rezeptur vorhandenen Inhaltstoffe;
         und
      Bereitstellen zumindest eines Produktionsattributs, betreffend den Massenanteil aerob leicht abbaubarer (Ready Biodegradable) Inhaltsstoffe;
      und/oder
   C6 Herstellen oder Bereitstellen der Inhaltsstoffe, vorzugsweise in jeweils getrennten Gefäßen, gemäß der in Schritt C1 bereitgestellten Rezeptur
      und
      Bestimmung des Massenanteils organischer Substanzen die in Untersuchungen gemäß OECD 311, Adopted 23. March 2006 (OECD GUIDELINE FOR TESTING OF CHEMICALS, Anaerobic Biodegradability of Organic Compounds in Digested Sludge: By Measurement of Gas Production) eine vollständige anerobe Biodegradation (complete anaerobic biodegradation) zeigen (Massenanteil anaerob abbaubarer Inhaltsstoffe) bezogen auf die Gesamtmasse der gemäß der in Schritt C1 bereitgestellten Rezeptur vorhandenen Inhaltstoffe;
         und
      Bereitstellen zumindest eines Produktionsattributs, betreffend den Anteil organsicher Substanzen, die in Untersuchungen gemäß OECD 311, Adopted 23. March 2006 (OECD GUIDELINE FOR TESTING OF CHEMICALS, Anaerobic Biodegradability of Organic Compounds in Digested Sludge: By Measurement of Gas Production) eine vollständige anerobe Biodegradation (complete anaerobic biodegradation) zeigen (Massenanteil anaerob abbaubarer Inhaltsstoffe);
      und/oder
   C7 Herstellen oder Bereitstellen der Inhaltsstoffe, vorzugsweise in jeweils getrennten Gefäßen, gemäß der in Schritt C1 bereitgestellten Rezeptur
      und
      Bestimmung des Massenanteils biologisch hochwertig kreislauffähiger Inhaltsstoffe, bezogen auf die Gesamtmasse der gemäß der in Schritt C1 bereitgestellten Rezeptur vorhandenen Inhaltstoffe;
         und
      Bereitstellen zumindest eines Produktionsattributs, betreffend den Massenanteil biologisch hochwertig kreislauffähiger Inhaltsstoffe;
      und/oder
   C8 Herstellen oder Bereitstellen der Inhaltsstoffe, vorzugsweise in jeweils getrennten Gefäßen, gemäß der in Schritt C1 bereitgestellten Rezeptur
      und
      Bestimmung des Massenanteils
         - der kombinierten Gesamtmasse aller Atomen, die aus nachwachsenden Rohstoffen oder aus der Luft stammen, vorzugsweise Gesamtmasse an Atomen ausgewählt aus der Gruppe bestehend aus:
            - Kohlenstoffatme,
            - Wasserstoffatome,
            - Sauerstoffatome,
               und
            - Stickstoffatome,
            die aus nachwachsenden Rohstoffen oder aus der Luft stammen
            an
         - der kombinierten Gesamtmasse sämtlicher organischer und anorganischer Inhaltstoffe außer Wasser der gemäß der in Schritt C1 bereitgestellten Rezeptur;
         und
      Bereitstellen zumindest eines Produktionsattributs, betreffend den vorstehend definierten Massenanteil;
      und
C9 Zuordnen von einem, zwei, drei, vier, fünf, sechs oder sieben der in den Schritten C2, C3, C4, C5, C6, C7 C8 und C9 bereitgestellten Produktionsattribute zu einem Ausgangsmaterialidentifikator oder mehreren Ausgangsmaterialidentifikatoren;
   und optional
C10 Berechnung einer optischen Darstellung und optische Darstellung des in Schritt C9 beteiligten Ausgangsmaterialidentifikators bzw. der in Schritt C9 beteiligten Ausgangsmaterialidentifikatoren, vorzugsweise als Spinnendiagramm.

Zu Verschlüssen zählen im Rahmen der vorliegenden Erfindung auch Verschlüsse von Dosierspendern also beispielsweise auch Dosierpumpen, Sprühaufsätze etc.

Aspekt A2: Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, bevorzugt wie vorstehend als bevorzugt bezeichnet, besonders bevorzugt gemäß vorstehendem Aspekt A1),
wobei das Verfahren zur Herstellung zumindest die folgenden Schritte umfasst:
D1 Bereitstellen einer Rezeptur für eine Primärverpackung eines Waschmittels und/oder eines Reinigungsmittels und/oder einer kosmetischen Zubereitung, umfassend zumindest einen Anteil an Rezyklat;
   und/oder
D2 Bereitstellen einer Rezeptur für einen Verschluss einer Primärverpackung eines Waschmittels und/oder eines Reinigungsmittels und/oder einer kosmetischen Zubereitung, umfassend zumindest einen Anteil an Rezyklat;
   und wobei das Verfahren zusätzlich zumindest einen, bevorzugt zumindest zwei, besonders bevorzugt zumindest drei, ganz besonders bevorzugt sämtliche der folgenden Schritte umfasst:
   D3 Herstellen oder Bereitstellen der Inhaltsstoffe, vorzugsweise in jeweils getrennten Gefäßen, gemäß der in Schritt D1 bereitgestellten Rezeptur
      und
      Bestimmung des Massenanteils an Post-Consumer Recycled Material (PCR) bezogen auf die Gesamtmasse der gemäß der in Schritt D1 bereitgestellten Rezeptur vorhandenen Inhaltstoffe;
         und
      Bereitstellen zumindest eines Produktionsattributs, betreffend den entsprechenden Massenanteil an Post-Consumer Recycled Material (PCR);
      und/oder
   D4 Herstellen oder Bereitstellen der Inhaltsstoffe, vorzugsweise in jeweils getrennten Gefäßen, gemäß der in Schritt D2 bereitgestellten Rezeptur
      und
      Bestimmung des Massenanteils an Post-Consumer Recycled Material (PCR) bezogen auf die Gesamtmasse der gemäß der in Schritt D2 bereitgestellten Rezeptur vorhandenen Inhaltstoffe;
         und
      Bereitstellen zumindest eines Produktionsattributs, betreffend den entsprechenden Massenanteil an Post-Consumer Recycled Material (PCR);
      und/oder
   D5 Herstellen oder Bereitstellen der Inhaltsstoffe, vorzugsweise in jeweils getrennten Gefäßen, gemäß der in Schritt D1 bereitgestellten Rezeptur
      und
      Herstellen oder Bereitstellen der Inhaltsstoffe, vorzugsweise in jeweils getrennten Gefäßen, gemäß der in Schritt D2 bereitgestellten Rezeptur
         und
      Bestimmung des Massenanteils an Post-Consumer Recycled Material (PCR) bezogen auf die kombinierte Gesamtmasse der gemäß den in Schritten D1 und D2 bereitgestellten Rezepturen vorhandenen Inhaltstoffe;
         und
      Bereitstellen zumindest eines Produktionsattributs, betreffend den entsprechenden Massenanteil an Post-Consumer Recycled Material (PCR);
      und/oder
   D6 Herstellen oder Bereitstellen der Inhaltsstoffe, vorzugsweise in jeweils getrennten Gefäßen, gemäß der in Schritt D1 bereitgestellten Rezeptur
      und
      Bestimmung des kombinierten Massenanteils an ungefärbten und weißen Kunststoffkomponenten bezogen auf die Gesamtmasse der gemäß der in Schritt D1 bereitgestellten Rezeptur vorhandenen Inhaltstoffe;
         und
      Bereitstellen zumindest eines Produktionsattributs, betreffend den entsprechenden kombinierten Massenanteil an ungefärbten und weißen Kunststoffkomponenten;
      und/oder
   D7 Herstellen oder Bereitstellen der Inhaltsstoffe, vorzugsweise in jeweils getrennten Gefäßen, gemäß der in Schritt D2 bereitgestellten Rezeptur
      und
      Bestimmung des kombinierten Massenanteils an ungefärbten und weißen Kunststoffkomponenten bezogen auf die Gesamtmasse der gemäß der in Schritt D2 bereitgestellten Rezeptur vorhandenen Inhaltstoffe;
         und
      Bereitstellen zumindest eines Produktionsattributs, betreffend den entsprechenden kombinierten Massenanteil an ungefärbten und weißen Kunststoffkomponenten;
      und/oder
   D8 Herstellen oder Bereitstellen der Inhaltsstoffe, vorzugsweise in jeweils getrennten Gefäßen, gemäß der in Schritt D1 bereitgestellten Rezeptur
      und
      Herstellen oder Bereitstellen der Inhaltsstoffe, vorzugsweise in jeweils getrennten Gefäßen, gemäß der in Schritt D2 bereitgestellten Rezeptur
         und
      Bestimmung des kombinierten Massenanteils an ungefärbten und weißen Kunststoffkomponenten bezogen auf die kombinierte Gesamtmasse der gemäß den in Schritten D1 und D2 bereitgestellten Rezepturen vorhandenen Inhaltstoffe;
         und
      Bereitstellen zumindest eines Produktionsattributs, betreffend den entsprechenden kombinierten Massenanteil an ungefärbten und weißen Kunststoffkomponenten;
      und/oder
   D9 Herstellen oder Bereitstellen der Inhaltsstoffe, vorzugsweise in jeweils getrennten Gefäßen, gemäß der in Schritt D1 bereitgestellten Rezeptur
      und
      Bestimmung des Massenanteils an mechanisch recycelbaren Materialien bezogen auf die Gesamtmasse der gemäß der in Schritt D1 bereitgestellten Rezeptur vorhandenen Inhaltstoffe;
         und
      Bereitstellen zumindest eines Produktionsattributs, betreffend den entsprechenden Massenanteil an mechanisch recycelbaren Materialien;
      und/oder
   D10 Herstellen oder Bereitstellen der Inhaltsstoffe, vorzugsweise in jeweils getrennten Gefäßen, gemäß der in Schritt D2 bereitgestellten Rezeptur
      und
      Bestimmung des Massenanteils an mechanisch recycelbaren Materialien bezogen auf die Gesamtmasse der gemäß der in Schritt D2 bereitgestellten Rezeptur vorhandenen Inhaltstoffe;
         und
      Bereitstellen zumindest eines Produktionsattributs, betreffend den entsprechenden Massenanteil an mechanisch recycelbaren Materialien;
      und/oder
   D11 Herstellen oder Bereitstellen der Inhaltsstoffe, vorzugsweise in jeweils getrennten Gefäßen, gemäß der in Schritt D1 bereitgestellten Rezeptur
      und
      Herstellen oder Bereitstellen der Inhaltsstoffe, vorzugsweise in jeweils getrennten Gefäßen, gemäß der in Schritt D2 bereitgestellten Rezeptur
         und
      Bestimmung des Massenanteils an mechanisch recycelbaren Materialien bezogen auf die kombinierte Gesamtmasse der gemäß den in Schritten D1 und D2 bereitgestellten Rezepturen vorhandenen Inhaltstoffe;
         und
      Bereitstellen zumindest eines Produktionsattributs, betreffend den entsprechenden Massenanteil an mechanisch recycelbaren Materialien;
      und/oder
   D12 Herstellen oder Bereitstellen der Inhaltsstoffe, vorzugsweise in jeweils getrennten Gefäßen, gemäß der in Schritt D1 bereitgestellten Rezeptur
      und
      Bestimmung des Massenanteils an mechanisch qualitätserhaltend recycelbaren Materialien bezogen auf die Gesamtmasse der gemäß der in Schritt D1 bereitgestellten Rezeptur vorhandenen Inhaltstoffe;
         und
      Bereitstellen zumindest eines Produktionsattributs, betreffend den entsprechenden Massenanteil an mechanisch qualitätserhaltend recycelbaren Materialien;
      und/oder
   D13 Herstellen oder Bereitstellen der Inhaltsstoffe, vorzugsweise in jeweils getrennten Gefäßen, gemäß der in Schritt D2 bereitgestellten Rezeptur
      und
      Bestimmung des Massenanteils an mechanisch qualitätserhaltend recycelbaren Materialien bezogen auf die Gesamtmasse der gemäß der in Schritt D2 bereitgestellten Rezeptur vorhandenen Inhaltstoffe;
         und
      Bereitstellen zumindest eines Produktionsattributs, betreffend den entsprechenden Massenanteil an mechanisch qualitätserhaltend recycelbaren Materialien;
      und/oder
   D14 Herstellen oder Bereitstellen der Inhaltsstoffe, vorzugsweise in jeweils getrennten Gefäßen, gemäß der in Schritt D1 bereitgestellten Rezeptur
      und
      Herstellen oder Bereitstellen der Inhaltsstoffe, vorzugsweise in jeweils getrennten Gefäßen, gemäß der in Schritt D2 bereitgestellten Rezeptur
         und
      Bestimmung des Massenanteils an mechanisch qualitätserhaltend recycelbaren Materialien bezogen auf die kombinierte Gesamtmasse der gemäß den in Schritten D1 und D2 bereitgestellten Rezepturen vorhandenen Inhaltstoffe;
         und
      Bereitstellen zumindest eines Produktionsattributs, betreffend den entsprechenden Massenanteil an mechanisch qualitätserhaltend recycelbaren Materialien;
      und
D15 Zuordnen von einem, zwei, drei, vier, fünf, sechs, sieben, acht, neun, zehn, elf oder zwölf der in den Schritten D3, D4, D5, D6, D7, D8, D9, D10, D11, D12, D13 und D14 bereitgestellten Produktionsattribute zu einem Ausgangsmaterialidentifikator oder mehreren Ausgangsmaterialidentifikatoren;
   und optional
D16 Berechnung einer optischen Darstellung und optische Darstellung des in Schritt D15 beteiligten Ausgangsmaterialidentifikators bzw. der in Schritt D15 beteiligten Ausgangsmaterialidentifikatoren, vorzugsweise als Spinnendiagramm.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei
- die chemische Zubereitung ein Waschmittel und/oder ein Reinigungsmittel und/oder eine kosmetische Zubereitung ist;
   und/oder
- wobei die Verpackung nebst Verschluss vorzugsweise nebst Verschluss und Etikett, für die chemische Zubereitung eine Verpackung nebst Verschluss vorzugsweise nebst Verschluss und Etikett, für ein Waschmittel und/oder für ein Reinigungsmittel und/oder für eine kosmetische Zubereitung ist.

Das Verfahren wird in diesen Fällen besonderes effizient durchgeführt. Insbesondere werden auf diese Weise Produkte mit besonders vorteilhaften Ausgangsmaterialidentifikatoren hergestellt.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet),
- wobei der eine Eingangsmaterialstrom betreffend die chemische Zubereitung oder die mehreren Eingangsmaterialströme betreffend die chemische Zubereitung in das chemische Produktionsnetzwerk an einem oder mehreren Eingangspunkten einer oder mehrerer chemischer Produktionsketten eintreten, wobei das chemische Produktionsnetzwerk eine oder mehrere chemische Produktionsketten umfasst;
   und/oder
- wobei der eine Eingangsmaterialstrom betreffend die Verpackung nebst Verschluss vorzugsweise nebst Verschluss und Etikett, oder die mehreren Eingangsmaterialströme betreffend die Verpackung nebst Verschluss vorzugsweise nebst Verschluss und Etikett, in das chemische Produktionsnetzwerk an einem oder mehreren Eingangspunkten einer oder mehrerer chemischer Produktionsketten eintreten, wobei das chemische Produktionsnetzwerk eine oder mehrere chemische Produktionsketten umfasst.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei sich der Recyclingmaterialtyp auf mindestens eine Eigenschaft des Recyclingmaterialstroms bezieht, wobei die Eigenschaft eine Materialzusammensetzung des Recyclingmaterials, eine Herkunft des Recyclingmaterials, einen Herkunftstyp des Recyclingmaterials, eine Recyclingstufe des Recyclingmaterials und/oder eine Verwendung des Recyclingmaterials während seines Lebenszyklus umfasst.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei das Zuweisen mindestens eines Produktionsattributs das Erzeugen von Ausgleichseinheiten für das eine oder die mehreren Produktionsattribute, das Auswählen mindestens eines Materialkontos, das mit einem oder mehreren Produktionsattributen verbunden ist, die einem oder mehreren Zielattributen entsprechen, und das Bereitstellen von Ausgleichseinheiten für das mindestens eine Materialkonto umfasst.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei das Zuweisen mindestens eines Produktionsattributs das Bereitstellen eines oder mehrerer Zielattribute, die dem Ausgangsmaterial zugeordnet werden sollen, und das Auswählen mindestens eines Materialkontos, das einem oder mehreren Produktionsattributen zugeordnet ist, die einem oder mehreren Zielattributen entsprechen, umfasst.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei dem chemischen Produktionsnetzwerk mehrere Eingangsmaterialströme zugeführt werden, die mit einem oder mehreren Produktionsattributen verbunden sind.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei ein oder mehrere Eingangsstoffströme dem chemischen Produktionsnetzwerk an verschiedenen Eingangspunkten des chemischen Produktionsnetzes zugeführt werden.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei die Zuordnung mindestens eines Produktionsattributs zum Ausgangsmaterialidentifikator die Bestimmung des/der zur Herstellung des Ausgangsmaterials verwendeten EingangsmaterialstromsZ-ströme und die Auswahl mindestens eines Materialkontos umfasst, das mit dem/den Produktionsattribut/en verbunden ist, die sich auf den/die bestimmten Eingangsmaterialstrom/-ströme beziehen.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei die Zuordnung mindestens eines Produktionsattributs zum Ausgangsmaterialidentifikator die Bestimmung des/der zur Herstellung des Ausgangsmaterials verwendeten Eingangsmaterialstroms/Eingangsmaterialströme und des Beitrags des/der bestimmten Eingangsmaterialstroms/Eingangsmaterialströme zum Ausgangsmaterial umfasst.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet), wobei sich mindestens ein Produktionsattribut auf ein Umweltattribut bezieht, wobei das Umweltattribut die Umweltauswirkungen des (der) Eingangsmaterialstroms (-ströme) oder des (der) Ausgangsmaterials (-ströme) digital spezifiziert.

Bevorzugt ist ein Verfahren zur Herstellung einer chemischen Zubereitung in einer Verpackung, nebst Verschluss, vorzugsweise nebst Verschluss und Etikett, für eine chemische Zubereitung (wie vorstehend beschrieben, vorzugsweise wie vorstehend als bevorzugt bezeichnet),
- wobei die Eingangsmaterialdaten betreffend Eingangsmaterialien für die chemische Zubereitung mehrere Eingangsmaterialströme betreffend die chemische Zubereitung umfassen
   und
   wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das mit dem Gesamtanteil von tensidischen und nicht-tensidischen ¹⁴C-Atomen verbunden ist, welcher einem Alter der entsprechenden Kohlenstoffatome von weniger als 300 Jahren seit der Kohlenstoffdioxid-Fixierung entspricht
      und
   wobei das zumindest eine Produktionsattribut dem ersten Ausgangsmaterialidentifikator betreffend die chemische Zubereitung zugeordnet wird;
   und/oder
- wobei die Eingangsmaterialdaten betreffend Eingangsmaterialien für die chemische Zubereitung mehrere Eingangsmaterialströme betreffend die chemische Zubereitung umfassen
   und
   wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das der Pflanzenart verbunden ist aus der Anteile des Eingangsmaterialstrom stammen
      und
   wobei das zumindest eine Produktionsattribut dem ersten Ausgangsmaterialidentifikator betreffend die chemische Zubereitung zugeordnet wird;
   und/oder
- wobei die Eingangsmaterialdaten betreffend Eingangsmaterialien für die chemische Zubereitung mehrere Eingangsmaterialströme betreffend die chemische Zubereitung umfassen
   und
   wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das mit dem Massenanteil aerob leicht abbaubar Substanzen im Eingangsmaterialstrom verknüpft ist
      und
   wobei das zumindest eine Produktionsattribut dem ersten Ausgangsmaterialidentifikator betreffend die chemische Zubereitung zugeordnet wird;
   und/oder
- wobei die Eingangsmaterialdaten betreffend Eingangsmaterialien für die chemische Zubereitung mehrere Eingangsmaterialströme betreffend die chemische Zubereitung umfassen
   und
   wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das mit dem Massenanteil anaerob abbaubar Substanzen im Eingangsmaterialstrom verknüpft ist
      und
   wobei das zumindest eine Produktionsattribut dem ersten Ausgangsmaterialidentifikator betreffend die chemische Zubereitung zugeordnet wird;
   und/oder
- wobei die Eingangsmaterialdaten betreffend Eingangsmaterialien für die chemische Zubereitung mehrere Eingangsmaterialströme betreffend die chemische Zubereitung umfassen
   und
   wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das mit dem Massenanteil biologisch hochwertig kreislauffähiger Materialien im Eingangsmaterialstrom verknüpft ist
      und
   wobei das zumindest ein Produktionsattribut dem ersten Ausgangsmaterialidentifikator betreffend die chemische Zubereitung zugeordnet wird;
   und/oder
- wobei die Eingangsmaterialdaten betreffend Eingangsmaterialien für die Verpackung mehrere Eingangsmaterialströme betreffend die Verpackung umfassen
   und
   wobei zumindest einem Eingangsmaterialstrom betreffend die Verpackung oder mehreren der Eingangsmaterialströme betreffend die Verpackung zumindest ein Produktionsattribut zugeordnet ist, das mit dem Massenanteil mechanisch rezyklierbarer Materialien im Eingangsmaterialstrom verknüpft ist
      und
   wobei das zumindest ein Produktionsattribut dem zweiten Ausgangsmaterialidentifikator betreffend die Verpackung zugeordnet wird;
   und/oder
- wobei die Eingangsmaterialdaten betreffend Eingangsmaterialien für die Verpackung mehrere Eingangsmaterialströme betreffend die Verpackung umfassen
   und
   wobei zumindest einem Eingangsmaterialstrom betreffend die Verpackung oder mehreren der Eingangsmaterialströme betreffend die Verpackung zumindest ein Produktionsattribut zugeordnet ist, das mit dem Massenanteil mechanisch upcyclingfähiger Materialien im Eingangsmaterialstrom verknüpft ist
      und
   wobei das zumindest ein Produktionsattribut dem zweiten Ausgangsmaterialidentifikator betreffend die Verpackung zugeordnet wird;
   und/oder
- wobei die Eingangsmaterialdaten betreffend Eingangsmaterialien für die Verpackung mehrere Eingangsmaterialströme betreffend die Verpackung umfassen
   und
   wobei zumindest einem Eingangsmaterialstrom betreffend die Verpackung oder mehreren der Eingangsmaterialströme betreffend die Verpackung zumindest ein Produktionsattribut zugeordnet ist, das mit dem kombinierten Massenanteil ungefärbter und weißer Kunststoffkomponenten im Eingangsmaterialstrom verknüpft ist
      und
   wobei das zumindest ein Produktionsattribut dem zweiten Ausgangsmaterialidentifikator betreffend die Verpackung zugeordnet wird;
   und/oder
- wobei die Eingangsmaterialdaten betreffend Eingangsmaterialien für den Verschluss mehrere Eingangsmaterialströme betreffend den Verschluss umfassen
   und
   wobei zumindest einem Eingangsmaterialstrom betreffend den Verschluss oder mehreren der Eingangsmaterialströme betreffend den Verschluss zumindest ein Produktionsattribut zugeordnet ist, das mit dem Massenanteil mechanisch rezyklierbarer Materialien im Eingangsmaterialstrom verknüpft ist
      und
   wobei das zumindest ein Produktionsattribut dem dritten Ausgangsmaterialidentifikator betreffend den Verschluss zugeordnet wird;
   und/oder
- wobei die Eingangsmaterialdaten betreffend Eingangsmaterialien für den Verschluss mehrere Eingangsmaterialströme betreffend den Verschluss umfassen
   und
   wobei zumindest einem Eingangsmaterialstrom betreffend den Verschluss oder mehreren der Eingangsmaterialströme betreffend den Verschluss zumindest ein Produktionsattribut zugeordnet ist, das mit dem Massenanteil mechanisch upcyclingfähiger Materialien im Eingangsmaterialstrom verknüpft ist
      und
   wobei das zumindest ein Produktionsattribut dem dritten Ausgangsmaterialidentifikator betreffend den Verschluss zugeordnet wird;
   und/oder
- wobei die Eingangsmaterialdaten betreffend Eingangsmaterialien für den Verschluss mehrere Eingangsmaterialströme betreffend den Verschluss umfassen
   und
   wobei zumindest einem Eingangsmaterialstrom betreffend den Verschluss oder mehreren der Eingangsmaterialströme betreffend den Verschluss zumindest ein Produktionsattribut zugeordnet ist, das mit dem kombinierten Massenanteil ungefärbter und weißer Kunststoffkomponenten im Eingangsmaterialstrom verknüpft ist
      und
   wobei das zumindest ein Produktionsattribut dem dritten Ausgangsmaterialidentifikator betreffend den Verschluss zugeordnet wird.

In vielen Fällen werden vorzugsweise im Verfahren Eingangsmaterialströme umfassend Tenside ausgeschlossen bzw. deren Einsatz minimiert, bei denen nicht Anteile der Tenside im Eingangsmaterialstrom aus einer Pflanze stammen, vorzugsweise nicht Anteile der Tenside im Eingangsmaterialstrom aus einer Pflanzenart stammen, die zur nachfolgend definierten Gruppe von Pflanzen gehört: Hanf, Haselnuss, Distel, Erdnuss, Erdmandel, Jojoba, Iberischer Drachenkopf, Leindotter, Flachs, Mandel, Mohn, Nachtkerze, Olive, Ölrettich, Ölrauke, Pekannuss, Pistazie, Raps, Rizinus, Rübsen, Saflor, Schwarzkümmel, Sesam, Sonnenblume, Soja, Walnuss, Weintraube und Kombinationen daraus.

Eine weitere Aufgabe der vorliegenden Erfindung wird gelöst durch eine Vorrichtung zur Überwachung einer oder mehrerer Produktionseigenschaften eines Produktionsmaterials, das von einem chemischen Produktionsnetzwerk produziert wird, wobei die Vorrichtung Folgendes umfasst:
- eine Datenbereitstellungsschnittstelle, die so konfiguriert ist, dass sie Eingangsmaterialdaten, die mit einem oder mehreren Eingangsmaterialströmen und einem oder mehreren zugehörigen Produktionsattributen assoziiert sind, an eine Computerschnittstelle liefert, wobei der eine oder die mehreren Eingangsmaterialströme an das chemische Produktionsnetzwerk geliefert werden;
- eine Zuweisungseinheit, die so konfiguriert ist, dass sie das/die eine(n) Produktionsattribut(e) mindestens einem Materialkonto zuweist, das mit dem/den jeweiligen Produktionsattribut(en) verbunden ist, und zwar auf der Grundlage des/der einen oder mehreren Produktionsattributs/e, das/die mit dem/den einen oder mehreren Eingangsmaterialstrom/en verbunden ist/sind,

- eine Identifizierungseinheit, die so konfiguriert ist, dass sie ein Ausgangsmaterialidentifikator bereitstellt, der mit dem von dem chemischen Produktionsnetzwerk produzierten Ausgangsmaterial verbunden ist,
- eine Zuordnungseinheit, die so konfiguriert ist, dass sie mindestens ein Produktionsattribut aus dem mindestens einen Materialkonto, das mit dem jeweiligen Produktionsattribut verbunden ist, dem mindestens einen Ausgangsmaterialbezeichner zuordnet.

Eine weitere Aufgabe der vorliegenden Erfindung wird gelöst durch ein Verfahren zur Herstellung mindestens eines Ausgangsmaterials, das mit einem oder mehreren Produktionsattributen verbunden ist, wobei das mindestens eine Ausgangsmaterial durch ein chemisches Produktionsnetzwerk hergestellt wird, wobei das Verfahren umfasst:
- Bereitstellung eines Eingangsmaterialstroms oder mehrerer Eingangsmaterialströme, die mit einem oder mehreren Produktionsattributen verbunden sind, an einem oder mehreren Eingangspunkten des chemischen Produktionsnetzes,
- Bereitstellen von Eingangsmaterialdaten, die mit einem oder mehreren Eingangsmaterialströmen verbunden sind und mit einem oder mehreren Produktionsattributen verbunden sind, an eine Computerschnittstelle, wobei der eine oder die mehreren Eingangsmaterialströme dem chemischen Produktionsnetzwerk bereitgestellt werden;
- auf der Grundlage des/der einen oder mehreren Produktionsattributs/e, das/die mit dem/den einen oder mehreren Eingangsmaterialstrom/en verbunden ist/sind, Zuweisung des/der einen oder mehreren Produktionsattributs/e zu mindestens einem Materialkonto, das mit dem/den jeweiligen Produktionsattribut/en verbunden ist,
- Bereitstellung eines Ausgangsmaterialidentifikators, der mit dem vom chemischen Produktionsnetzwerk produzierten Ausgangsmaterial verbunden ist,
- Herstellung des mindestens einen Ausgangsmaterials und Bereitstellung des Ausgangsmaterials an einem oder mehreren Ausgangspunkten des chemischen Produktionsnetzes,
- Zuordnen mindestens eines Produktionsattributs aus dem mindestens einen Materialkonto, das mit dem jeweiligen Produktionsattribut verbunden ist, zu dem mindestens einen Ausgangsmaterialidentifikator.
- Bereitstellung des mindestens einen Ausgabematerials, das mit dem Ausgabematerialidentifikator und dem zugewiesenen mindestens einen Produktionsattribut verbunden ist.

Eine weitere Aufgabe der vorliegenden Erfindung wird gelöst durch ein System zur Herstellung mindestens eines Ausgangsmaterials, das mit einem oder mehreren Produktionsattributen verbunden ist, wobei das mindestens eine Ausgangsmaterial durch ein chemisches Produktionsnetzwerk hergestellt wird, wobei das System umfasst:
- ein chemisches Produktionsnetz, das so konfiguriert ist, dass es einen oder mehrere Eingangsmaterialströme, die mit einem oder mehreren Produktionsattributen verbunden sind, an einem oder mehreren Eingangspunkten des chemischen Produktionsnetzes empfängt, um das mindestens eine Ausgangsmaterial aus dem einen oder den mehreren Eingangsmaterialströmen herzustellen und das Ausgangsmaterial an einem oder mehreren Ausgangspunkten des chemischen Produktionsnetzes bereitzustellen,
- eine Produktionsbetriebsvorrichtung, die zur Überwachung eines Eingangsmaterialstroms oder mehrerer Eingangsmaterialströme, assoziiert mit einer Produktionsbetriebsvorrichtung, die so konfiguriert ist, dass sie eine oder mehrere Produktionseigenschaften des Ausgangsmaterials überwacht, das von dem chemischen Produktionsnetzwerk gemäß einem der Verfahren (wie vorstehend beschrieben, vorzugsweise wie vorstehend als bevorzugt bezeichnet),
wobei das System so konfiguriert ist, dass es mindestens ein Ausgabematerial bereitstellt, das mit dem Ausgabematerialidentifikator und dem mindestens einen zugewiesenen Produktionsattribut verbunden ist.

## Patentansprüche

1. Verfahren zur Herstellung einer chemischen Zubereitung in einer Verpackung, nebst Verschluss, für eine chemische Zubereitung,
umfassend ein computerimplementiertes Verfahren zur Überwachung eines oder mehrerer Produktionsattribute eines von einem chemischen Produktionsnetzwerk produzierten Ausgangsmaterials, wobei das Verfahren, vorzugsweise das computerimplementierte Verfahren, die folgenden Schritte umfasst:
S1a Bereitstellung von Eingangsmaterialdaten betreffend Eingangsmaterialien für die chemische Zubereitung, die
- mit einem Eingangsmaterialstrom betreffend die chemische Zubereitung oder mehreren Eingangsmaterialströmen betreffend die chemische Zubereitung
und
- mit einem oder mehreren Produktionsattributen betreffend die chemische Zubereitung
verbunden sind, an eine Computerschnittstelle,
wobei der eine Eingangsmaterialstrom betreffend die chemische Zubereitung oder die mehreren Eingangsmaterialströme betreffend die chemische Zubereitung dem chemischen Produktionsnetzwerk zugeführt werden
und
wobei mindestens ein Eingangsmaterialstrom ein Tensid umfasst, das einen Anteil an ¹⁴C-Atomen umfasst, welcher einem Alter der entsprechenden Kohlenstoffatome von weniger als 300 Jahren seit der Kohlenstoffdioxid-Fixierung entspricht;
wobei dem einen Eingangsmaterialstrom betreffend die chemische Zubereitung oder den mehreren Eingangsmaterialströmen betreffend die chemische Zubereitung zumindest ein Produktionsattribut zugeordnet ist, das mit dem Anteil an ¹⁴C-Atomen verknüpft ist;
S2a basierend auf dem einen Produktionsattribut das mit dem einen Eingangsmaterialstrom betreffend die chemische Zubereitung verbunden ist oder auf den mehreren Produktionsattributen, die mit den mehreren Eingangsmaterialströmen betreffend die chemische Zubereitung verbunden sind:
Zuordnen des einen Produktionsattributs betreffend die chemische Zubereitung oder der mehreren Produktionsattribute betreffend die chemische Zubereitung zu mindestens einem Materialkonto, das mit dem jeweiligen Produktionsattribut verbunden ist;
S3a Bereitstellung eines Ausgangsmaterialidentifikators, der mit dem vom chemischen Produktionsnetzwerk produzierten Ausgangsmaterial verbunden ist;
S4a Zuordnen
- mindestens eines Produktionsattributs aus dem mindestens einen Materialkonto das mit dem jeweiligen Produktionsattribut zugeordneten Produktionsattribut verbunden ist,
zu einem ersten Ausgangsmaterialidentifikator betreffend die chemische Zubereitung.

2. Verfahren zur Herstellung einer chemischen Zubereitung in einer Verpackung, nebst Verschluss, vorzugsweise nebst Verschluss und Etikett, für eine chemische Zubereitung nach Anspruch 1,
wobei das computerimplementierte Verfahren zusätzlich die folgenden Schritte umfasst:
S1b Bereitstellung von Eingangsmaterialdaten betreffend Eingangsmaterialien für die Verpackung, die
- mit einem Eingangsmaterialstrom betreffend die Verpackung oder mehreren Eingangsmaterialströmen betreffend die Verpackung
und
- mit einem oder mehreren Produktionsattributen betreffend die Verpackung
verbunden sind, an eine Computerschnittstelle,
wobei der eine Eingangsmaterialstrom betreffend die Verpackung oder die mehreren Eingangsmaterialströme betreffend die Verpackung dem chemischen Produktionsnetzwerk zugeführt werden
und
wobei mindestens ein Eingangsmaterialstrom betreffend die Verpackung ein Post-Consumer Recycled Material umfasst;
wobei dem einen Eingangsmaterialstrom betreffend die Verpackung oder den mehreren Eingangsmaterialströmen betreffend die Verpackung zumindest ein Produktionsattribut zugeordnet ist, das mit dem Anteil an Post-Consumer Recycled Material verknüpft ist;
S2b basierend auf dem einen Produktionsattribut, das mit dem einen Eingangsmaterialstrom verbunden ist oder auf den mehreren Produktionsattributen, die mit den mehreren Eingangsmaterialströmen verbunden sind:
Zuordnen des einen Produktionsattributs betreffend die Verpackung oder der mehreren Produktionsattribute betreffend die Verpackung zu mindestens einem Materialkonto, das mit dem jeweiligen Produktionsattribut verbunden ist;
S3b Bereitstellung eines Ausgangsmaterialidentifikators, der mit dem vom chemischen Produktionsnetzwerk produzierten Ausgangsmaterial verbunden ist;
S4b Zuordnen
- mindestens eines Produktionsattributs betreffend die Verpackung aus dem mindestens einen Materialkonto, das mit dem jeweiligen Produktionsattribut zugeordneten Produktionsattribut verbunden ist,
zu einem zweiten Ausgangsmaterialidentifikator betreffend die Verpackung.

3. Verfahren zur Herstellung einer chemischen Zubereitung in einer Verpackung, nebst Verschluss, vorzugsweise nebst Verschluss und Etikett, für eine chemische Zubereitung nach einem der vorangehenden Ansprüche,
wobei das computerimplementierte Verfahren zusätzlich die folgenden Schritte umfasst:
S1c Bereitstellung von Eingangsmaterialdaten betreffend Eingangsmaterialien für den Verschluss, die
- mit einem Eingangsmaterialstrom betreffend den Verschluss oder mehreren Eingangsmaterialströmen betreffend den Verschluss
und
- mit einem oder mehreren Produktionsattributen betreffend den Verschluss
verbunden sind, an eine Computerschnittstelle,
wobei der eine Eingangsmaterialstrom betreffend den Verschluss oder die mehreren Eingangsmaterialströme betreffend den Verschluss dem chemischen Produktionsnetzwerk zugeführt werden
und
wobei mindestens ein Eingangsmaterialstrom betreffend den Verschluss einen Anteil an Post-Consumer Recycled Material umfasst;
wobei dem einen Eingangsmaterialstrom betreffend den Verschluss oder den mehreren Eingangsmaterialströmen betreffend den Verschluss zumindest ein Produktionsattribut zugeordnet ist, das mit dem Anteil an Post-Consumer Recycled Material verknüpft ist;
S2c basierend auf dem einen Produktionsattribut, das mit dem einen Eingangsmaterialstrom verbunden ist oder auf den mehreren Produktionsattributen, die mit den mehreren Eingangsmaterialströmen verbunden sind:
Zuordnen des einen Produktionsattributs betreffend den Verschluss oder der mehreren Produktionsattribute betreffend den Verschluss zu mindestens einem Materialkonto, das mit dem jeweiligen Produktionsattribut verbunden ist;
S3c Bereitstellung eines Ausgangsmaterialidentifikators, der mit dem vom chemischen Produktionsnetzwerk produzierten Ausgangsmaterial verbunden ist;
S4c Zuordnen
- mindestens eines Produktionsattributs betreffend den Verschluss aus dem mindestens einen Materialkonto, das mit dem jeweiligen Produktionsattribut zugeordneten Produktionsattribut verbunden ist,
zu einem dritten Ausgangsmaterialidentifikator betreffend den Verschluss.

4. Verfahren zur Herstellung einer chemischen Zubereitung in einer Verpackung, nebst Verschluss und Etikett, für eine chemische Zubereitung nach einem der vorangehenden Ansprüche,
wobei die Verpackung eine Verpackung mit einem daran angebrachten Etikett ist und
wobei das computerimplementierte Verfahren zusätzlich die folgenden Schritte umfasst:
S1d Bereitstellung von Eingangsmaterialdaten betreffend Eingangsmaterialien für das Etikett, die
- mit einem Eingangsmaterialstrom betreffend das Etikett oder mehreren Eingangsmaterialströmen betreffend das Etikett
und
- mit einem oder mehreren Produktionsattributen betreffend das Etikett
verbunden sind, an eine Computerschnittstelle,
wobei der eine Eingangsmaterialstrom betreffend das Etikett oder die mehreren Eingangsmaterialströme betreffend das Etikett dem chemischen Produktionsnetzwerk zugeführt werden
und
wobei mindestens ein Eingangsmaterialstrom betreffend das Etikett einen Anteil an Post-Consumer Recycled Material umfasst;
wobei dem einen Eingangsmaterialstrom betreffend das Etikett oder den mehreren Eingangsmaterialströmen betreffend das Etikett zumindest ein Produktionsattribut zugeordnet ist, das mit dem Anteil an Post-Consumer Recycled Material verknüpft ist;
S2d basierend auf dem einen Produktionsattribut, das mit dem einen Eingangsmaterialstrom betreffend das Etikett verbunden ist oder auf den mehreren Produktionsattributen, die mit den mehreren Eingangsmaterialströmen betreffend das Etikett verbunden sind:
Zuordnen des einen Produktionsattributs betreffend das Etikett oder der mehreren Produktionsattribute betreffend das Etikett zu mindestens einem Materialkonto, das mit dem jeweiligen Produktionsattribut verbunden ist;
S3d Bereitstellung eines Ausgangsmaterialidentifikators, der mit dem vom chemischen Produktionsnetzwerk produzierten Ausgangsmaterial umfassend das Etikett verbunden ist;
S4d Zuordnen
- mindestens eines Produktionsattributs betreffend das Etikett aus dem mindestens einen Materialkonto, das mit dem jeweiligen Produktionsattribut zugeordneten Produktionsattribut verbunden ist,
zu einem vierten Ausgangsmaterialidentifikator betreffend das Etikett.

5. Verfahren nach einem der vorangehenden Ansprüche, wobei das computerimplementierte Verfahren zusätzlich den folgenden Schritt umfasst:
S5 Miteinanderverrechnen von zwei, drei oder sämtlichen Ausgangsmaterialindikatoren, ausgewählt aus der Gruppe bestehend aus:
- erster Ausgangsmaterialidentifikator betreffend die chemische Zubereitung, resultierend in Schritt S4a;
und
- zweiter Ausgangsmaterialidentifikator betreffend die Verpackung, resultierend in Schritt S4b;
und
- dritter Ausgangsmaterialidentifikator betreffend den Verschluss, resultierend in Schritt S4c;
und
- vierter Ausgangsmaterialidentifikator betreffend das Etikett, resultierend in Schritt S4d;
so dass ein Ausgangsmaterialidentifikator betreffend ein Konsumprodukt resultiert.

6. Verfahren nach einem der vorangehenden Ansprüche, wobei
- die chemische Zubereitung ein Waschmittel und/oder ein Reinigungsmittel und/oder eine kosmetische Zubereitung ist;
und/oder
- wobei die Verpackung nebst Verschluss vorzugsweise nebst Verschluss und Etikett, für die chemische Zubereitung eine Verpackung nebst Verschluss vorzugsweise nebst Verschluss und Etikett, für ein Waschmittel und/oder für ein Reinigungsmittel und/oder für eine kosmetische Zubereitung ist.

7. Verfahren nach einem der vorangehenden Ansprüche,
- wobei der eine Eingangsmaterialstrom betreffend die chemische Zubereitung oder die mehreren Eingangsmaterialströme betreffend die chemische Zubereitung in das chemische Produktionsnetzwerk an einem oder mehreren Eingangspunkten einer oder mehrerer chemischer Produktionsketten eintreten, wobei das chemische Produktionsnetzwerk eine oder mehrere chemische Produktionsketten umfasst;
und/oder
- wobei der eine Eingangsmaterialstrom betreffend die Verpackung nebst Verschluss oder die mehreren Eingangsmaterialströme betreffend die Verpackung nebst Verschluss in das chemische Produktionsnetzwerk an einem oder mehreren Eingangspunkten einer oder mehrerer chemischer Produktionsketten eintreten, wobei das chemische Produktionsnetzwerk eine oder mehrere chemische Produktionsketten umfasst.

8. Verfahren nach einem der vorhergehenden Ansprüche,
- wobei sich der Recyclingmaterialtyp auf mindestens eine Eigenschaft des Recyclingmaterialstroms bezieht, wobei die Eigenschaft eine Materialzusammensetzung des Recyclingmaterials, eine Herkunft des Recyclingmaterials, einen Herkunftstyp des Recyclingmaterials, eine Recyclingstufe des Recyclingmaterials und/oder eine Verwendung des Recyclingmaterials während seines Lebenszyklus umfasst;
und/oder
- wobei das Zuweisen mindestens eines Produktionsattributs das Erzeugen von Ausgleichseinheiten für das eine oder die mehreren Produktionsattribute, das Auswählen mindestens eines Materialkontos, das mit einem oder mehreren Produktionsattributen verbunden ist, die einem oder mehreren Zielattributen entsprechen, und das Bereitstellen von Ausgleichseinheiten für das mindestens eine Materialkonto umfasst;
und/oder
- wobei das Zuweisen mindestens eines Produktionsattributs das Bereitstellen eines oder mehrerer Zielattribute, die dem Ausgangsmaterial zugeordnet werden sollen, und das Auswählen mindestens eines Materialkontos, das einem oder mehreren Produktionsattributen zugeordnet ist, die einem oder mehreren Zielattributen entsprechen, umfasst;
und/oder
- wobei dem chemischen Produktionsnetzwerk mehrere Eingangsmaterialströme zugeführt werden, die mit einem oder mehreren Produktionsattributen verbunden sind;
und/oder
- wobei ein oder mehrere Eingangsstoffströme dem chemischen Produktionsnetzwerk an verschiedenen Eingangspunkten des chemischen Produktionsnetzes zugeführt werden.

9. Verfahren nach einem der vorhergehenden Ansprüche, wobei die Zuordnung mindestens eines Produktionsattributs zum Ausgangsmaterialidentifikator die Bestimmung des/der zur Herstellung des Ausgangsmaterials verwendeten Eingangsmaterialstroms/-ströme und die Auswahl mindestens eines Materialkontos umfasst, das mit dem/den Produktionsattribut/en verbunden ist, die sich auf den/die bestimmten Eingangsmaterialstrom/-ströme beziehen.

10. Verfahren nach einem der vorhergehenden Ansprüche, wobei die Zuordnung mindestens eines Produktionsattributs zum Ausgangsmaterialidentifikator die Bestimmung des/der zur Herstellung des Ausgangsmaterials verwendeten Eingangsmaterialstroms/Eingangsmaterialströme und des Beitrags des/der bestimmten Eingangsmaterialstroms/Eingangsmaterialströme zum Ausgangsmaterial umfasst.

11. Verfahren nach einem der vorhergehenden Ansprüche, wobei sich mindestens ein Produktionsattribut auf ein Umweltattribut bezieht, wobei das Umweltattribut die Umweltauswirkungen des Eingangsmaterialstroms/der Eingangsmaterialströme oder des Ausgangsmaterials/der Ausgangsmaterialströme digital spezifiziert.

12. Verfahren nach einem der vorangehenden Ansprüche,
- wobei die Eingangsmaterialdaten betreffend Eingangsmaterialien für die chemische Zubereitung mehrere Eingangsmaterialströme betreffend die chemische Zubereitung umfassen und
wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das mit dem Gesamtanteil von tensidischen und nicht-tensidischen ¹⁴C-Atomen verbunden ist, welcher einem Alter der entsprechenden Kohlenstoffatome von weniger als 300 Jahren seit der Kohlenstoffdioxid-Fixierung entspricht
und
wobei das zumindest eine Produktionsattribut dem ersten Ausgangsmaterialidentifikator betreffend die chemische Zubereitung zugeordnet wird;
und/oder
- wobei die Eingangsmaterialdaten betreffend Eingangsmaterialien für die chemische Zubereitung mehrere Eingangsmaterialströme betreffend die chemische Zubereitung umfassen
und
wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das der Pflanzenart verbunden ist aus der Anteile des Eingangsmaterialstrom stammen
und
wobei das zumindest eine Produktionsattribut dem ersten Ausgangsmaterialidentifikator betreffend die chemische Zubereitung zugeordnet wird;
und/oder
- wobei die Eingangsmaterialdaten betreffend Eingangsmaterialien für die chemische Zubereitung mehrere Eingangsmaterialströme betreffend die chemische Zubereitung umfassen
und
wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das mit dem Massenanteil aerob leicht abbaubar Substanzen im Eingangsmaterialstrom verknüpft ist
und
wobei das zumindest eine Produktionsattribut dem ersten Ausgangsmaterialidentifikator betreffend die chemische Zubereitung zugeordnet wird;
und/oder
- wobei die Eingangsmaterialdaten betreffend Eingangsmaterialien für die chemische Zubereitung mehrere Eingangsmaterialströme betreffend die chemische Zubereitung umfassen
und
wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das mit dem Massenanteil anaerob abbaubar Substanzen im Eingangsmaterialstrom verknüpft ist
und
wobei das zumindest eine Produktionsattribut dem ersten Ausgangsmaterialidentifikator betreffend die chemische Zubereitung zugeordnet wird;
und/oder
- wobei die Eingangsmaterialdaten betreffend Eingangsmaterialien für die chemische Zubereitung mehrere Eingangsmaterialströme betreffend die chemische Zubereitung umfassen
und
wobei zumindest einem Eingangsmaterialstrom betreffend die chemische Zubereitung oder mehreren der Eingangsmaterialströme betreffend die chemische Zubereitung zumindest ein Produktionsattribut zugeordnet ist, das mit dem Massenanteil biologisch hochwertig kreislauffähiger Materialien im Eingangsmaterialstrom verknüpft ist
und
wobei das zumindest ein Produktionsattribut dem ersten Ausgangsmaterialidentifikator betreffend die chemische Zubereitung zugeordnet wird;
und/oder
- wobei die Eingangsmaterialdaten betreffend Eingangsmaterialien für die Verpackung mehrere Eingangsmaterialströme betreffend die Verpackung umfassen
und
wobei zumindest einem Eingangsmaterialstrom betreffend die Verpackung oder mehreren der Eingangsmaterialströme betreffend die Verpackung zumindest ein Produktionsattribut zugeordnet ist, das mit dem Massenanteil mechanisch rezyklierbarer Materialien im Eingangsmaterialstrom verknüpft ist
und
wobei das zumindest ein Produktionsattribut dem zweiten Ausgangsmaterialidentifikator betreffend die Verpackung zugeordnet wird;
und/oder
- wobei die Eingangsmaterialdaten betreffend Eingangsmaterialien für die Verpackung mehrere Eingangsmaterialströme betreffend die Verpackung umfassen
und
wobei zumindest einem Eingangsmaterialstrom betreffend die Verpackung oder mehreren der Eingangsmaterialströme betreffend die Verpackung zumindest ein Produktionsattribut zugeordnet ist, das mit dem Massenanteil mechanisch upcyclingfähiger Materialien im Eingangsmaterialstrom verknüpft ist
und
wobei das zumindest ein Produktionsattribut dem zweiten Ausgangsmaterialidentifikator betreffend die Verpackung zugeordnet wird;
und/oder
- wobei die Eingangsmaterialdaten betreffend Eingangsmaterialien für die Verpackung mehrere Eingangsmaterialströme betreffend die Verpackung umfassen
und
wobei zumindest einem Eingangsmaterialstrom betreffend die Verpackung oder mehreren der Eingangsmaterialströme betreffend die Verpackung zumindest ein Produktionsattribut zugeordnet ist, das mit dem kombinierten Massenanteil ungefärbter und weißer Kunststoffkomponenten im Eingangsmaterialstrom verknüpft ist
und
wobei das zumindest ein Produktionsattribut dem zweiten Ausgangsmaterialidentifikator betreffend die Verpackung zugeordnet wird;
und/oder
- wobei die Eingangsmaterialdaten betreffend Eingangsmaterialien für den Verschluss mehrere Eingangsmaterialströme betreffend den Verschluss umfassen
und
wobei zumindest einem Eingangsmaterialstrom betreffend den Verschluss oder mehreren der Eingangsmaterialströme betreffend den Verschluss zumindest ein Produktionsattribut zugeordnet ist, das mit dem Massenanteil mechanisch rezyklierbarer Materialien im Eingangsmaterialstrom verknüpft ist
und
wobei das zumindest ein Produktionsattribut dem dritten Ausgangsmaterialidentifikator betreffend den Verschluss zugeordnet wird;
und/oder
- wobei die Eingangsmaterialdaten betreffend Eingangsmaterialien für den Verschluss mehrere Eingangsmaterialströme betreffend den Verschluss umfassen
und
wobei zumindest einem Eingangsmaterialstrom betreffend den Verschluss oder mehreren der Eingangsmaterialströme betreffend den Verschluss zumindest ein Produktionsattribut zugeordnet ist, das mit dem Massenanteil mechanisch upcyclingfähiger Materialien im Eingangsmaterialstrom verknüpft ist
und
wobei das zumindest ein Produktionsattribut dem dritten Ausgangsmaterialidentifikator betreffend den Verschluss zugeordnet wird;
und/oder
- wobei die Eingangsmaterialdaten betreffend Eingangsmaterialien für den Verschluss mehrere Eingangsmaterialströme betreffend den Verschluss umfassen
und
wobei zumindest einem Eingangsmaterialstrom betreffend den Verschluss oder mehreren der Eingangsmaterialströme betreffend den Verschluss zumindest ein Produktionsattribut zugeordnet ist, das mit dem kombinierten Massenanteil ungefärbter und weißer Kunststoffkomponenten im Eingangsmaterialstrom verknüpft ist
und
wobei das zumindest ein Produktionsattribut dem dritten Ausgangsmaterialidentifikator betreffend den Verschluss zugeordnet wird.

13. Eine Vorrichtung zur Überwachung einer oder mehrerer Produktionseigenschaften eines Produktionsmaterials, das von einem chemischen Produktionsnetzwerk produziert wird, wobei die Vorrichtung Folgendes umfasst:
- eine Datenbereitstellungsschnittstelle, die so konfiguriert ist, dass sie Eingangsmaterialdaten, die mit einem oder mehreren Eingangsmaterialströmen und einem oder mehreren zugehörigen Produktionsattributen assoziiert sind, an eine Computerschnittstelle liefert, wobei der eine oder die mehreren Eingangsmaterialströme an das chemische Produktionsnetzwerk geliefert werden;
- eine Zuweisungseinheit, die so konfiguriert ist, dass sie das eine Produktionsattribut / die mehreren Produktionsattribute mindestens einem Materialkonto zuweist, das mit dem jeweiligen Produktionsattribut/ den jeweiligen Produktionsattributen verbunden ist, und zwar auf der Grundlage des/der einen oder mehreren Produktionsattributs/e, das/die mit dem/den einen oder mehreren Eingangsmaterialstrom/en verbunden ist/sind,
- eine Identifizierungseinheit, die so konfiguriert ist, dass sie ein Ausgangsmaterialidentifikator bereitstellt, der mit dem von dem chemischen Produktionsnetzwerk produzierten Ausgangsmaterial verbunden ist,
- eine Zuordnungseinheit, die so konfiguriert ist, dass sie mindestens ein Produktionsattribut aus dem mindestens einen Materialkonto, das mit dem jeweiligen Produktionsattribut verbunden ist, dem mindestens einen Ausgangsmaterialbezeichner zuordnet.

14. Verfahren zur Herstellung mindestens eines Ausgangsmaterials, das mit einem oder mehreren Produktionsattributen verbunden ist, wobei das mindestens eine Ausgangsmaterial durch ein chemisches Produktionsnetzwerk hergestellt wird, wobei das Verfahren umfasst:
- Bereitstellung eines Eingangsmaterialstroms oder mehrerer Eingangsmaterialströme, die mit einem oder mehreren Produktionsattributen verbunden sind, an einem oder mehreren Eingangspunkten des chemischen Produktionsnetzes,
- Bereitstellen von Eingangsmaterialdaten, die mit einem oder mehreren Eingangsmaterialströmen verbunden sind und mit einem oder mehreren Produktionsattributen verbunden sind, an eine Computerschnittstelle, wobei der eine oder die mehreren Eingangsmaterialströme dem chemischen Produktionsnetzwerk bereitgestellt werden;
- auf der Grundlage des/der einen oder mehreren Produktionsattributs/e, das/die mit dem/den einen oder mehreren Eingangsmaterialstrom/en verbunden ist/sind, Zuweisung des/der einen oder mehreren Produktionsattributs/e zu mindestens einem Materialkonto, das mit dem/den jeweiligen Produktionsattribut/en verbunden ist,
- Bereitstellung eines Ausgangsmaterialidentifikators, der mit dem vom chemischen Produktionsnetzwerk produzierten Ausgangsmaterial verbunden ist,
- Herstellung des mindestens einen Ausgangsmaterials und Bereitstellung des Ausgangsmaterials an einem oder mehreren Ausgangspunkten des chemischen Produktionsnetzes,
- Zuordnen mindestens eines Produktionsattributs aus dem mindestens einen Materialkonto, das mit dem jeweiligen Produktionsattribut verbunden ist, zu dem mindestens einen Ausgangsmaterialidentifikator.
- Bereitstellung des mindestens einen Ausgabematerials, das mit dem Ausgabematerialidentifikator und dem zugewiesenen mindestens einen Produktionsattribut verbunden ist.

15. System zur Herstellung mindestens eines Ausgangsmaterials, das mit einem oder mehreren Produktionsattributen verbunden ist, wobei das mindestens eine Ausgangsmaterial durch ein chemisches Produktionsnetzwerk hergestellt wird, wobei das System umfasst:
- ein chemisches Produktionsnetz, das so konfiguriert ist, dass es einen oder mehrere Eingangsmaterialströme, die mit einem oder mehreren Produktionsattributen verbunden sind, an einem oder mehreren Eingangspunkten des chemischen Produktionsnetzes empfängt, um das mindestens eine Ausgangsmaterial aus dem einen oder den mehreren Eingangsmaterialströmen herzustellen und das Ausgangsmaterial an einem oder mehreren Ausgangspunkten des chemischen Produktionsnetzes bereitzustellen,
- eine Produktionsbetriebsvorrichtung, die zur Überwachung eines Eingangsmaterialstroms oder mehrerer Eingangsmaterialströme, assoziiert mit einer Produktionsbetriebsvorrichtung, die so konfiguriert ist, dass sie eine oder mehrere Produktionseigenschaften des Ausgangsmaterials überwacht, das von dem chemischen Produktionsnetzwerk gemäß einem der Verfahren nach einem der Ansprüche 1 bis 12 oder von der Vorrichtung nach Anspruch 13 produziert wird,
wobei das System so konfiguriert ist, dass es das mindestens ein Ausgabematerial bereitstellt, das mit dem Ausgabematerialidentifikator und dem mindestens einen zugewiesenen Produktionsattribut verbunden ist.
